# EUROPEAN PATENT SPECIFICATION

(11) **EP 1 745 286 B1**
(45) Date of publication and mention of the grant of the patent: **03.03.2010**
(21) Application number: 05747435.5
(22) Date of filing: 11.05.2005
(51) Int. Cl.: G01N 33/50, C12M 1/12, C12M 1/34, C12M 3/06

(54) **CELL PERMEABILITY ASSAY IN A LIVING ARRAY OF MULTIPLE CELL TYPES AND MULTIPLE LAYERS OF A POROUS SUBSTRATE**
ZELLPERMEABILITÄTSTEST AUF EINEM LEBENDEN ARRAY BESTEHEND AUS MULTIPLEN ZELLTYPEN UND MEHREREN SCHICHTEN EINES PORÖSEN SUBSTRATS
ANALYSE DE LA PERMEABILITE CELLULAIRE DANS UN RESEAU VIVANT DE TYPES CELLULAIRES MULTIPLES ET PLUSIEUR COUCHES D'UN SUBSTRAT POREUX

(30) Priority: 12.05.2004 EP 04447118
(43) Date of publication of application: 24.01.2007
(73) Proprietor: PamGene B.V., 5211 's Hertogenbosch (NL)
(72) Inventor: INGHAM, Colin, John, NL-6707 DW Wageningen (NL); VAN ALEWIJK, Dirk, NL-4751 JS Oud-Gastel (NL)
(74) Representative: De Clercq, Ann G. Y.
(86) International application number: PCT/EP2005/005084
(87) International publication number: WO 2005/108983

(56) References cited:
- US-B1- 6 605 638
- RUBAS WERNER ET AL: "An integrated method to determine epithelial transport and bioactivity of oral drug candidates in vitro" PHARMACEUTICAL RESEARCH (NEW YORK), vol. 13, no. 1, 1996, pages 23-26, XP002298149 ISSN: 0724-8741
- CECCHELLI R ET AL: "In vitro model for evaluating drug transport across the blood-brain barrier" ADVANCED DRUG DELIVERY REVIEWS, vol. 36, no. 2-3, 5 April 1999 (1999-04-05), pages 165-178, XP002298150 ISSN: 0169-409X

## Description

The present invention relates to a device and use thereof for determining the efficacy of transporting drugs including pharmaceutical compositions across biological barriers, such as the Blood-Brain Barrier (BBB).

### Background Art

Passage of molecules across cells may be effected via passive diffusion, e.g. paracellular transport or transcellular transport. In addition, the body comprises biological barriers which separate different compartments or parts, and provide selected passage of molecules. Known biological barriers are for instance the blood-brain barrier, the placental barrier, the gastro-intestinal barrier, the skin and the blood testis barrier. Passage across biological barriers may be effected via carrier mediated transport or hindered by P-glycoprotein mediated efflux. Biological barriers may contribute to the permeability barrier between lumenal and interstitial compartments. Biological barriers may comprise tight junctions, or *zonulae occludentes,* which are specialized membrane domains that seal the intercellular spaces in epithelia and endothelia. Tight junctions not only separate distinct physiological compartments, but they also confer selectivity to the flux of molecules and ions through the intercellular spaces between the cells, the so-called paracellular pathway. In addition, tight junctions limit the diffusion of lipids between the apical and basolateral plasma membrane domains. Transcellular transport is the transport of compounds through the cell, i.e. compounds are absorbed on the apical side of the cell and released on the basolateral side.

The capillary endothelial cells of the brain transport not only nutrients but also drugs to the brain via various transport systems expressed in cell membranes. The transport system is collectively referred to as the "blood-brain barrier", e.g., a special physiological function which excretes metabolites of neurotransmitters and foreign materials from the brain to circulating blood, although, its physical existence is hardly elucidated. The blood-brain barrier (BBB) maintains a homeostatic environment in the central nervous system (CNS). The capillaries that supply the blood to the brain have tight junctions which block passage of most molecules through the capillary endothelial membranes. While the membranes do allow passage of various lipid soluble materials, such as heroin and other psychoactive drugs, water soluble materials such as glucose, proteins and amino acids do not pass through the BBB. Mediated transport mechanisms exist to transport glucose and essential amino acids across the BBB. Active transport mechanisms remove molecules which become in excess, such as potassium, from the brain. For a general review see Goldstein and Betz, 1986 and Betz et al, 1994. Evidence for the blood brain barrier is surmised from the knowledge that many drugs (foreign materials) are hardly transferred to the brain in spite of being highly lipid-soluble. The reason for this is considered to be in that drugs once transferred to the brain are excreted from the brain at dozens to hundreds as high as the transfer rate of the drugs. Accordingly, the blood-brain barrier plays a significantly important role in the transfer of a centrally acting drug. For example, the transfer of a therapeutic drug against AIDS virus, azidothymidine (AZT), into the brain is significantly limited due to the function of an excretion transport system in the blood-brain barrier (J. Pharmacol. Exp. Ther., 281: 369-375 (1997), ibid. 282: 1509-1517 (1997)).

Thus, the BBB impedes the delivery of some drugs to the CNS, while on the other hand some drugs are wrongly delivered to the CNS causing side-effects.

New drug discovery is an expensive and highly time-consuming process. Much of the expense is a result of the inability to quickly and easily determine whether a particular drug candidate (for instance an organic chemical compound) will be properly absorbed, distributed, metabolised, and excreted when administered to human patients.

If a compound is found to easily penetrate the blood-brain barrier, then it may be an effective neuro-active drug. But if that compound is not intended to be neuro-active, it may possess undesirable side effects such as drowsiness. Thus, the medicinal chemist should determine early in the drug development process whether or not a compound penetrates the blood-brain barrier.

Typically, the ADMET/PK properties (absorption, distribution, metabolism, excretion, toxicity/pharmacokinetic properties) of a given compound are difficult to predict. As a result, one must resort to expensive and time-consuming in vitro and in vivo experiments to provide the necessary information. Sometimes these experiments are rather unreliable, particularly in the case of relatively insoluble compounds. In the current state of drug development, many drug candidates travel rather far toward commercialisation before an intrinsic ADMET/PK flaw is discovered. The further such compounds travel down the development pathway, the more wasted expense they represent.

This problem is not new. To address it, medicinal chemists have traditionally employed various logical or algorithmic tools. Probably, the best known of the simple logical tools is Lipinski's Rule of 5. However, Lipinski's rule is still only a rough approximation. In addition, Lipinski's rule does not address the question whether a compound will or will not cross the blood-brain barrier, which is important in determining whether the drug will have significant side effects. Indeed, most models can not predict whether a compound would or would not penetrate the blood-brain barrier.

Methods have been designed to deliver needed drugs such as direct delivery within the CNS by intrathecal delivery which can be used with, for example, an Ommaya reservoir. United States Patent 5,455,044 provides for use of a dispersion system for CNS delivery or see United States Patent 5,558,852 for a discussion of other CNS delivery mechanisms as well as Betz et al. (1994) and Goldstein and Betz (1986).

There has been some progress in designing drugs that utilize the structure and function of the BBB itself to deliver the drugs. These drugs are designed to be lipid soluble or to be "piggy-backed" into the CNS by being coupled to peptides that can cross the BBB through mediated transport mechanisms. However, not all drugs are amenable to this solution. Pardridge and his colleagues have worked extensively in this area.

Pharmacological formulations that cross the blood-brain barrier can be administered. Such formulations can take advantage of methods now available to produce chimeric peptides in which a drug is coupled to a brain transport vector allowing transportation of these engineered drugs across the barrier (Pardridge, et al., 1992; Pardridge, 1992; Bickel, et al., 1993).

However, while these methods do provide CNS delivery for some drugs it would be useful to have additional means of testing in vitro the delivery of drugs to the CNS. An in vitro model for studying transport across the blood-brain-barrier is described in Checchelli et al, 1999 and US-A-6,605,638.

The problems encountered with the BBB, including impediment of drug delivery to the desired compartment or the wrong deliverance to a compartment, are likewise found in other systems having biological barriers, such as placental barriers, the gastro-intestinal barrier, the skin and the blood testis barrier.

Regarding placental barriers (PB), the general guideline in drug administration is that what the mother receives the fetus will receive through the placenta. The placenta is a vascular organ through which mother and fetus exchange materials by diffusion across the placental barrier. But transport and metabolism of drugs in the placenta are poorly understood because experimental studies on animals are difficult and on humans are impossible.

Another significant biological barrier providing selective passage is formed by the intestines. Absorption, for example, is highly relevant to medicinal chemists. If a particular compound cannot be easily absorbed within the intestine, then that compound will not be suitable for oral administration. Either the compound will have to be abandoned or other routes of administration will have to be considered.

What is needed therefore are additional simple and easy to use in vitro models for allowing the medicinal chemists to quickly discriminate between those compounds that have a relevant property of interest and those that do not, in particular whether said compounds reach there destination, e.g. by passing through biological barriers, where they then are supposed to exert their effect.

This invention pertains to models, devices and methods that test drugs relevant to medicinal chemists using a new device comprising barrier cells mimicking a biological barrier, e.g. the BBB.

### DESCRIPTION OF THE INVENTION

Throughout this disclosure, various publications, patents and published patent specifications are referenced by an identifying citation.

The practice of the present invention will employ, unless otherwise indicated, conventional techniques of organic chemistry, pharmacology, molecular biology (including recombinant techniques), cell biology, biochemistry, and immunology, which are within the skill of the art. Such techniques are explained fully in the literature, such as, "Molecular Cloning: A Laboratory Manual" Second Edition (Sambrook et al.,1989); "Animal Cell Culture" (R. I. Freshney, ed., 1987); the series "Methods in Enzymology" (Academic Press, Inc.); "Handbook of Experimental Immunology" (D. M. Weir & C. C. Blackwell, eds.); "Current Protocols in Molecular Biology" (F. M. Ausubel et al., eds., 1987, and periodic updates); and "Current Protocols in Immunology" (J. E. Coligan et al., eds., 1991).

As used herein, certain terms may have the following defined meanings. As used in the specification and claims, the singular form "a", "an" and "the" include plural references unless the context clearly dictates otherwise. For example, the term "a cell" includes a plurality of cells, including mixtures thereof.

As used herein, the term "comprising" is intended to mean that the products, compositions and methods include the recited elements, but not excluding others. "Consisting essentially of" when used to define products, compositions and methods, shall mean excluding other elements of any essential significance to the combination. Thus, a composition consisting essentially of the elements as defined herein would not exclude trace contaminants. "Consisting of" shall mean excluding more than trace elements of other ingredients and substantial method steps of the compositions of this invention.

Embodiments defined by each of these transition terms are within the scope of this invention. Before the present invention is described, it is to be understood that this invention is not limited to particular methods, components, or devices described, as such methods, components, and devices may, of course, vary. It is also to be understood that the terminology used herein is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein may be used in the practice or testing of the present invention, the preferred devices, methods and materials are now described.

### Device general

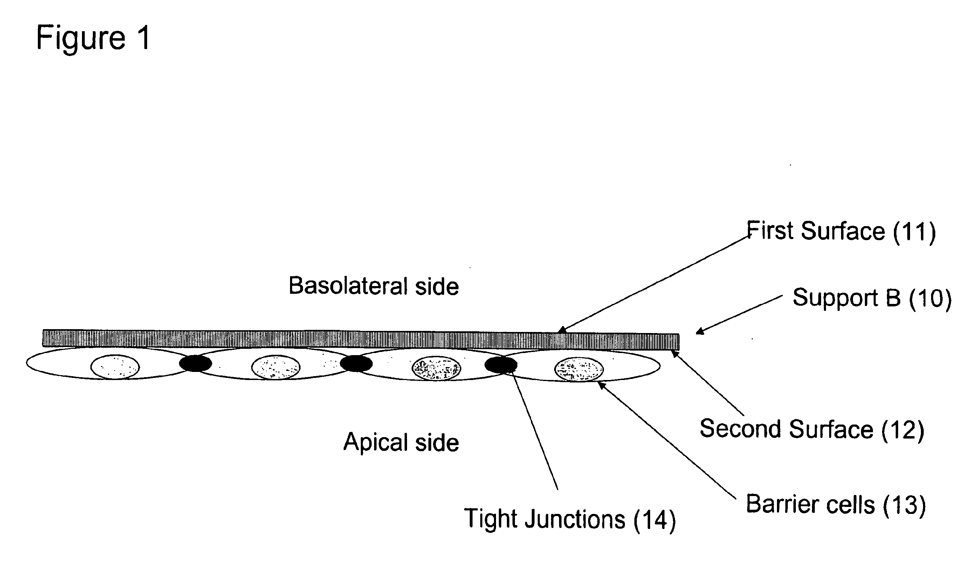

The present invention provides a device for determining drug efficacy on target cells comprising
(i) a porous support (B) having first and second surfaces and at least one area with a plurality of through-going channels, wherein said area comprises on said first surface at least one region, which can be contacted with target cells, and said area comprises on said second surface at least one region comprising a layer of barrier cells presenting a significant barrier adhered to said second surface, wherein said region on the first surface is located opposite to said region on the second surface of the porous support (B), and wherein said layer of barrier cells provides a barrier for the passage of drugs, and
(ii) a support (A) comprising drugs, wherein said support (A) can be contacted with the barrier cells of the porous support (B). Figure 1 provides a device according to the invention in which the layer of barrier cells presenting a significant barrier are barrier cells forming tight junctions.

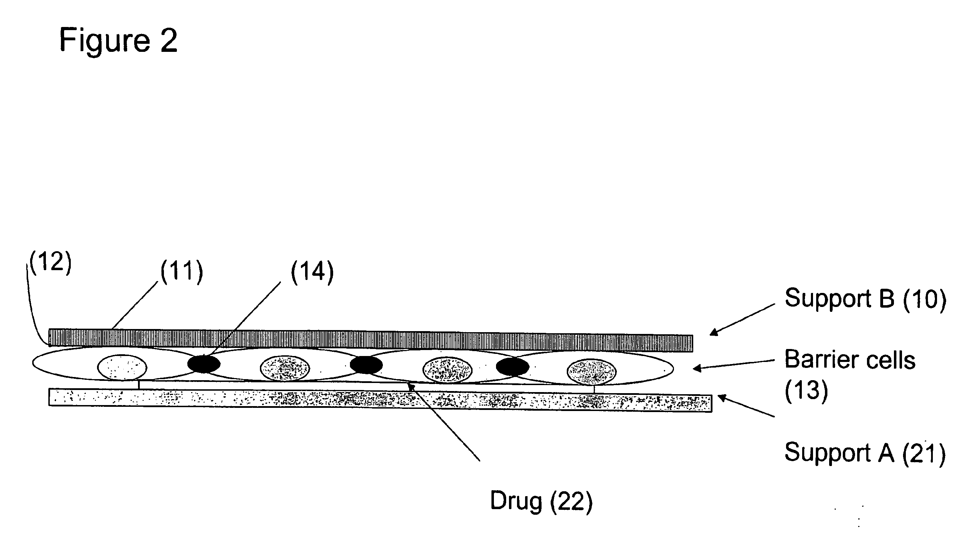

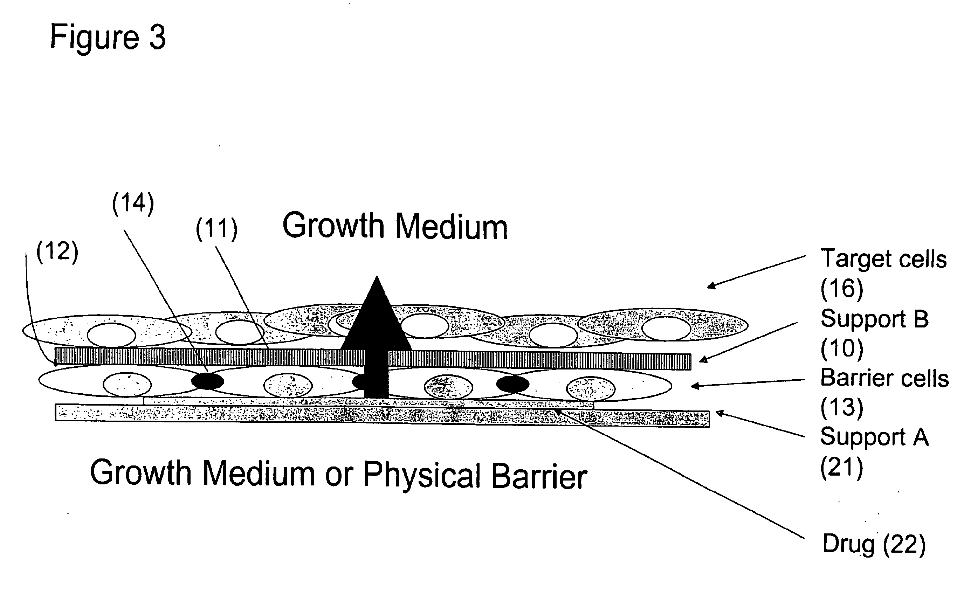

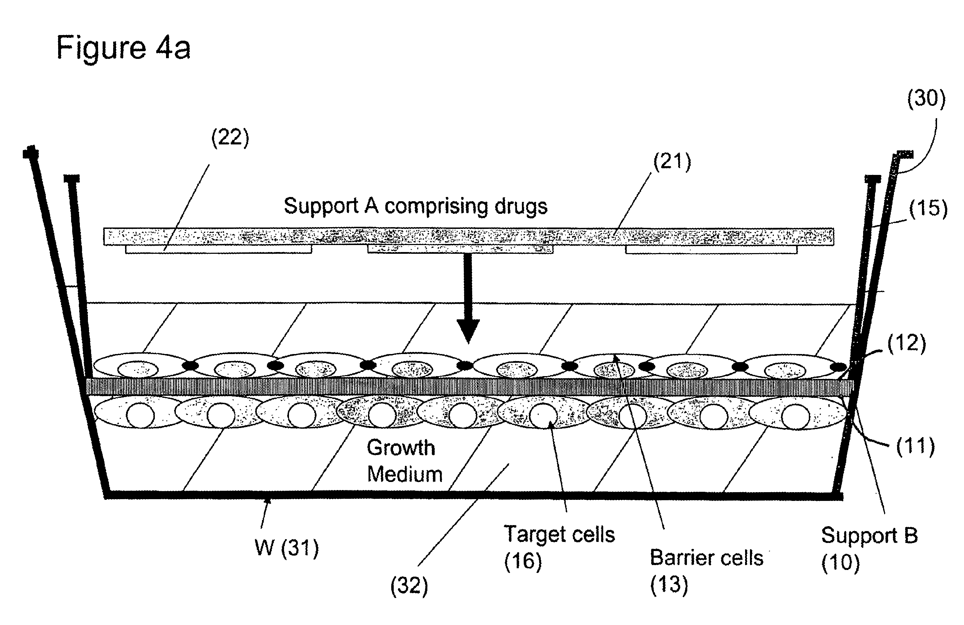

Accordingly, the present invention relates to the device as described herein, wherein said barrier cells presenting a significant barrier are barrier cells forming tight junctions.. In Figure 2 a support (A) is depicted comprising a drug which is brought into contact with a layer of barrier cells attached to the second surface of support (B). In an another embodiment, the device according to the invention further comprises target cells being in contact with a region on said first surface of porous support (B). It will be understood that not all target cells need to be in contact with said region on said first surface of porous support (B). Figure 3 depicts a device according to the invention comprising barrier cells forming tight junctions and attached to the second surface of support (B). A support (A) comprising a drug(s) contacts the barrier cells on the apical side. The drug releases from support (A), may pass through the barrier layer and porous support (B) and contacts the target cells, present on the first surface of support (B). Support (B) comprising the barrier cells may be placed in, e.g., a vial, chamber or container comprising growth medium. Figure 4A depicts an embodiment of the invention comprising of a porous support (B) comprising barrier cells which are attached to the second surface of the porous support (B) and target cells attached to its first surface, wherein said support (B) has edges (15) on its borders projecting from the second surface of support (B). This porous support (B) is placed into a chamber (30) with a bottom surface (W), with the basolateral side, i.e. the first surface, of the porous support (B) first. This chamber comprises growth medium (32). A support (A) comprising drugs (22) is depicted above the barrier cells, and may be placed onto the barrier cells whereby the drugs contact the barrier cells.

In a still further embodiment, the device according to the invention comprises a third support (W), which comprises target cells. In effect, the device according to the invention comprises:
(i) a support (W) comprising target cells;
(ii) a porous support (B) having first and second surfaces and at least one area with a plurality of through-going channels, wherein said area comprises on said second surface at least one region comprising a layer of barrier cells presenting a significant barrier and adhered to said second surface, and wherein the first surface of said porous support (B) can be contacted to the target cells of (i); and,
(iii) a support (A) comprising drugs, which can be contacted to said layer of barrier cells adhered to said second surface of the porous support (B).

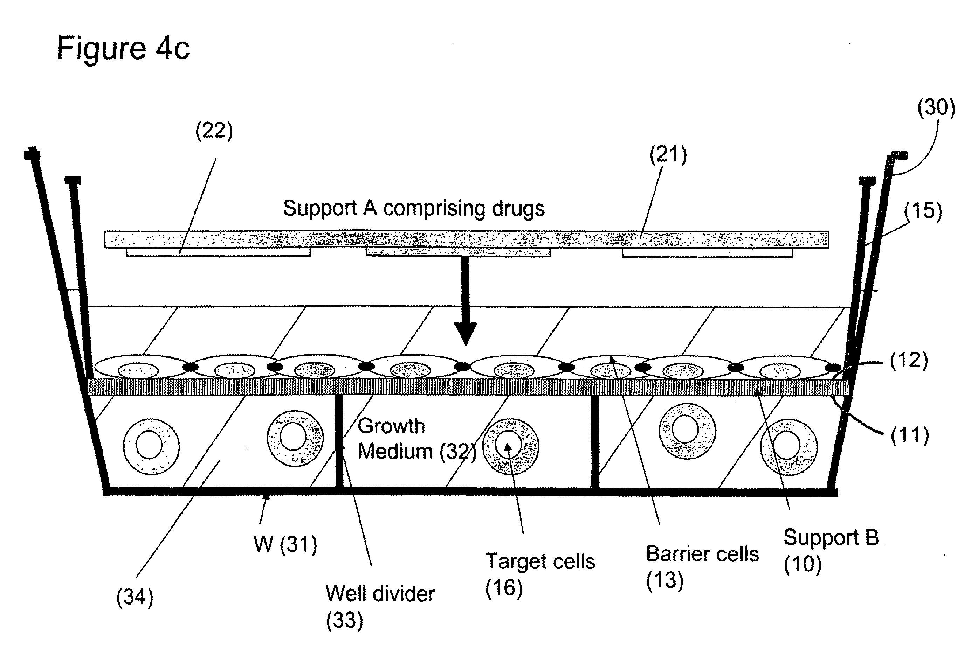

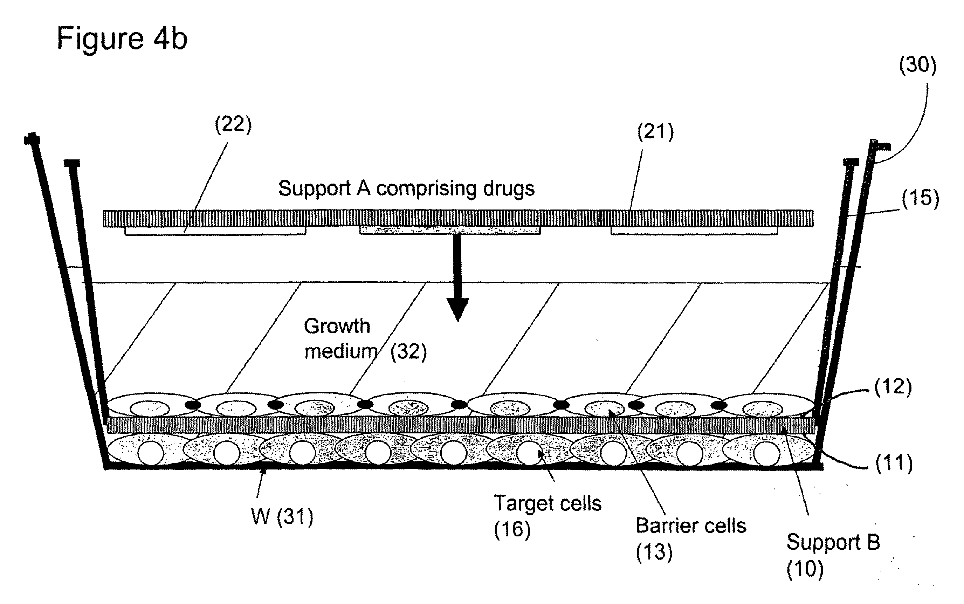

Figure 4B depicts a device according to the invention comprising of porous support (B) comprising barrier cells attached to its second surface. Support (B) has edges (15) on its borders projecting from the second surface of support (B). Support B may contact the chamber wall. The device further contains a chamber (30) with a bottom surface (W). Target cells are attached to the bottom surface (W) on the inside of the chamber. This chamber further comprises growth medium. Porous support (B) is placed into the chamber, with the basolateral side, i.e. the first surface, of the porous support (B) first. The first surface of support (B) may contact the target cells. Figure 4B also depicts a support (A) comprising drugs (22) above the barrier cells. Support (A) may be placed onto the barrier cells whereby the drugs (22) contact the barrier cells. In the device according to the invention, support (W) may comprise well dividers (33), which compartmentalize the target cells (see e.g. Figure 4C). The well dividers (33) define the borders of a well, which may comprise target cells. This is especially suitable in case of the target cells being non-adherent cells, e.g. cells in suspension. Figure 4C depicts a device according to the invention comprising a porous support (B) comprising barrier cells attached to its second surface. Support (B) has edges (15) on its borders projecting from the second surface of support (B). The device further contains a chamber (30) with a bottom surface (W), from which well dividers (33) protrude into the chamber. The wells contain target cells (16), which are in suspension, e.g. the target cells are suspended in growth medium (32). Porous support (B) is placed into the chamber, with the basolateral side, i.e. the first surface, of the porous support (B) first. The first surface of support (B) may contact the target cells physically, or via a fluid interface, e.g. the growth medium (32). Figure 4C also depicts a support (A) comprising drugs (22) above the barrier cells. Support (A) may be placed onto the barrier cells (13), whereby the drugs (22) contact the barrier cells (13). In this case, the surface of the drugs (22) spotted onto support (A) is smaller than the surface of the wells (34), defined by the well dividers (33) and the side parts of the chamber (30). In other words, a particular drug (22) is placed above and confined to one well (34) only. In other cases it may be convenient that the surface of a particular drug (22) or the drugs (22) is larger than the surface of the wells (34), and covers more than one well (34), e.g. 2 or 4 wells, for instance if the target cells (16) differ in the wells (34) covered by the drug (22).

It will be understood that in certain embodiments the barrier cells are located in between the porous support (B) and support (A). Both the porous support (B) and support (A) may contact the barrier cells, but on opposite sides of said barrier cells. As such, the present invention relates to a device as described herein, wherein said barrier cells are sandwiched between the porous support (B) and support (A)(Figure 2). In any event, if the barrier cells are sandwiched between the porous support (B) and support (A), then it must be ascertained that the drugs still can contact the barrier cells, e.g. the drugs are ' located on the internal side of support (A), and contact the barrier cells, or that support (A) is porous, in which case the drugs can pass through support (A).

In a further embodiment, the present invention relates to a sandwich micro array format, using at least two layers of a porous support and at least two cell types. This format is designed to test the penetration of a large number of potential drugs through cellular barriers, combined with an assay for the efficacy or toxicity of these drugs on one or more additional cell types.

### compartment

The first surface of the porous support (B) may be arrayed. The term "arrayed" refers to a support, and particularly a surface of a support, comprising particular, predefined regions and/or areas which are selectively addressable, and which have no or only minimally, e.g. not interfering with standard determination methods, exchange of components with adjacent regions and/or areas. The nature and geometry of the arrayed regions and/or areas will depend upon a variety of factors, including the intended use. For a given support size, the upper limit is determined only by the ability to create the arrays and detect a measurable signal in the device. The preferred number of arrays on a support generally depends on the particular use to which the device is to be put. In general, a support may contain from 2 to about 10⁴ arrayed regions and/or areas, or between about 10 and about 2000 arrayed regions and/or areas, or from about 20 to about 1000 arrayed regions and/or areas, or from 100 to 500 arrayed regions and/or areas, or about 300 arrayed regions and/or areas. Preferably, the number of the arrayed regions and/or areas relates preferentially to the number of standard SBS wells (Society for Biomolecular Screening (SBS) standard), or a part thereof, such as for instance, 1, 6, 24, 96, 386, or 1536. The arrayed regions and/or areas may be limited by physical borders, e.g. protruding from the surface, said borders forming a compartment. Accordingly, the present invention relates to a device as described herein, wherein said first surface of the porous support (B) comprises at least one compartment. The compartment may be located on the second surface of porous support (B), which comprises said layer of barrier cells adhered to said second surface. In addition, or in the alternative, said compartment may be located opposite to said region on the second surface of porous support (B) which comprises said layer of barrier cells adhered to said second surface. The compartment may be manufactured by any material standard in the art, such as described below for the housing. It will be understood that said compartment may comprise a liquid component, such as for instance a medium sustaining or allowing the survival or growth of cells. Accordingly, the present invention relates to a device as described herein, wherein said compartment is provided with a liquid medium comprising said target cells.

In a further embodiment, the device for determining drug efficacy on target cells comprises (i) a first micro scale chamber, comprising a support having drugs and on top thereof a first type of cell, under conditions where the first type of cell provides a significant barrier, e.g.; a layer with tight junctions, and (ii) a second micro scale chamber fitting into the first micro chamber, said second micro scale chamber comprising target cells and a porous support having first and second surfaces and at least one area with a plurality of through-going channels, wherein said area of said first surface is in contact with the target cells.

In a still further embodiment, the present invention relates to a porous support (B) having first and second surfaces and at least one area with a plurality of through-going channels, wherein said area comprises on said first surface at least one region, which can be contacted with target cells, and said area comprises on said second surface at least one region comprising a layer of barrier cells forming significant barrier, e.g. tight junctions, adhered to said second surface, wherein said region on the first surface is located opposite to said region on the second surface of the porous support (B), and wherein said layer of barrier cells provides a barrier for the passage of drugs.

### housing

The device of the invention may further comprise a housing into which the target cells, the barrier layer, the porous support (B), the support (A) and/or the support (W) are held. The housing may provide for several functions. Firstly, the housing may provide physical stability, i.e. it will be rigid, to the various components of the device. In particular, the housing may provide for holding the porous support (B), the support (A) and/or the support (W) at a predetermined location relative to each other and/or the barrier cells or target cells. As such, the housing may comprise one or various chambers. The various components of the device may releasably be placed in the housing. In this regard, the porous support (B), the support (A) and/or the support (W) may have regions intended for gripping, by e.g. forceps or tweezers. The housing may further provide for a controlled environment. Preferably, various environmental parameters may be controlled in the housing, such as for instance the temperature, atmosphere, including its concentration, pressure, humidity, and the like, which are parameters known by the person skilled in the art affecting the growth, sustainment, and viability of the barrier layer and/or the target cells. It will be understood that the chamber of the device may also provide the controlled environment. The housing of the device is preferably easily manufactured, in large amounts and cost-effectively, and may be made from materials standard in the art. In an embodiment, the material of which the housing body is made, is selected from the group consisting of glass, glass ceramics, plastic coated glass, metals, such as stainless steel, platinum, and/or aluminium, rubber, or plastics such as topaz, polypropylene, nylon, polyethylene, polystyrole, PET (polyethylene terephthalate), polycarbonate, silicones, polytetrafluor-ethylene, (teflon), polymethyl-methacrylate, and/or polymethane-ethylacrelate. The person skilled in the art will appreciate that other materials are feasible dependent on specific needs. Similarly, the chamber of the device may also be manufactured from the materials from which the housing body is made. Accordingly, the present invention relates to a device as described herein, further comprising a housing.

The present invention envisages the multiplex analysis of drug interactions with cells, whereby exposure of these cells to (a) one or more drugs, (b) drug concentration, and/or (c) one or more drugs in a function of time can be varied in a parallel manner. As such, support (A) of the device as described herein, comprises a supply chamber, which is attached to said support (A) and wherein said supply chamber comprises at least one compartment, which comprises one or more drugs. The compartments of the supply chamber control, e.g. vary, the deliverance of drug(s) to the barrier cells. Accordingly, the array of drugs is further arrayed.

### target cells

The target cells as described herein provide a means for determining the efficacy of drugs passing the layer of barrier cells, which present a significant barrier. As such, the target cells may be any cells, i.e. from any source, providing a measurable signal when affected by a drug. In other words, changes in the target cells due to the effect of the drug which has passed the significant barrier may be detected. In case of determining the efficacy drugs passing the BBB, cells of the central nervous system are preferred. In case of living target cells, the device provides an adequate representation of the natural situation. In addition; living cells provide intact and complete intracellular signalling pathways, encompassing micro-environmental influences from other parameters, such as changes of pH by the drugs. Accordingly, the present invention relates to a device as described herein, wherein said target cells are living target cells.

The present invention further relates to a device as described herein, wherein said target cells are recombinant cells, primary cells, or cells derived from a cell line. Preferably, the target cells are chosen from the group consisting of Caco-2 cells, MDCK cells, non-human embryonic stem cells, tumour cell lines, immobilised blood cells, including macrophages, or cells of the central nervous system.

In a number of cases, drugs are to be tested which are intended to treat pathogens of an individual. These pathogens may be intracellular or extracellular pathogens. Accordingly, in a further embodiment, the present invention relates to a device as described herein, wherein said target cells are tissue culture cells infected with an intracellular pathogen. In case of extracellular pathogens, or pathogens which may be existent outside the target cell, the target cell may be replaced by the extracellular pathogen itself, provided the drug efficacy can be measured on the extracellular pathogen, e.g. by killing the extracellular pathogen. Accordingly, in a further embodiment, the present invention relates to a device as described herein, wherein said target cell is replaced by a pathogen. Preferably, the intra- or extracellular pathogen is chosen from the group consisting of protozoa, fungi, bacteria, virus, viroids, prions and multicellular organisms.

The target cells may already be present in the device or may be added separately to the device before use. In an embodiment, the invention relates to a device as described herein, wherein said target cells are placed or grown on or recruited or adsorbed to said first surface of the porous support (B) or support (W). The target cells may artificially be localised to or tethered to said first surface of said porous support (B) or support (W), e.g. directed binding. Pre-treatment with compounds, such as for instance growth factors, gelatine, Matrigel (Becton-Dickinson), or other basement membrane components, such as collagens, laminin, entactin, proteoglycans, etc. may facilitate recruitment or adsorbance of the target cells to the support. Dependent on the growth of the target cells, possibly directed by the presence of further compounds on the first surface of support (B) or support (W), the target cells may be present in a layer which may be continuous or not, arrayed in rows and columns, discrete spots etc. The target cells may adhere to and cover, e.g. form a continuous layer on entirely said region as described above, and possibly on entirely said first surface of said porous support (B) or said support (W). In an alternative, the target cells are present at discrete locations on the first surface of support (B) or support (W), which may coincide with the locations and surfaces of drugs on support (A). As such, the target cells may be arrayed. For instance, the target cells may be located on particular regions and/or areas of the supports. Accordingly, the present invention relates to a device as described herein, wherein said target cells adhere to said first surface of said porous support (B).

Thus, the present invention relates also to a device as described herein, wherein said target cells are present in a layer, or are recruited by random or directed binding to the support. Preferably, the target cells are arrayed in rows and columns.

By virtue of the porosity of a porous support, it will be understood that the present invention relates to a device as described herein, wherein said target cells are comprised in and/or on the porous support (B), dependent on the pore size and the size of the target cells, possibly replaced by a pathogen or a component as defined below.

In an alternative, the target cells are in suspension, e.g. in the case of non-adherent cells. Accordingly, the present invention relates to a device as described herein, wherein said target cells are in suspension. In case of non-adherent cells, e.g. cells in suspension, the drugs may constitute a concentration gradient over the medium comprising the target cells. As such, the drugs have to cross a liquid interface before reaching the cells. Accordingly, the present invention relates to a device as described herein, wherein said target cells contact the first surface of said porous support (B) via a liquid interface.

The number of target cells is not limiting, but for practical considerations the maximum number of cells may be limited by the maximum number of cells which can be sustained, is able to grow, divide and/or differentiate, while the minimum number of target cells may be limited to the number of cells still able to generate a measurable signal after drug binding. Given the size of a porous support (B), a support (A) or a support (W) or the size of a region and/or area, the device as described herein may comprise at least about 1 to 10⁵ target cells, preferably about 10¹ to 10⁴ target cells, or preferably about 10² to 10³ target cells per region.

As mentioned above, the target cells provide a means for determining the efficacy of drugs passing the layer of barrier cells. As such, the target cells may be replaced by a component or by a fraction of a target cell, provided said component or said fraction generates a measurable signal when a drug binds to said component or said fraction. Accordingly, the present invention relates to a device as described herein, wherein said target cell is replaced by a component chosen from the group consisting of enzymes, enzyme substrates, peptides, oligo-peptides, proteins, receptors, ion-channels, lipids, carbohydrates, aptamers, ribozymes, nucleic acids, monoclonal or polyclonal antibodies, antibody fragments, and/or any derivatives and analogues thereof, or by a fraction of said target cell, such as an organelle, vesicle or membrane fraction. These components or fractions of a target cell provide ease in handling and storage compared to living target cells. In addition, these components or fractions of a target cell may provide ease in measuring, e.g. read-out, of drugs bound to these components and fractions. As such, the present invention relates to a device as described herein, wherein said component or fraction is immobilized by covalent attachment or adsorptive attachment to, in and/or on said porous support (B) or on said support (W), and possibly wherein said components or fractions are stored in dried or lyophilised condition on said porous support (B) or said support (W).

### barrier cells

The barrier cells as described herein are located on porous support (B). The barrier cells provide a significant barrier. A "significant barrier" is defined herein as a biological obstacle to the passage of drugs from one position to another position. A significant barrier may prevent free diffusion or other forms of transport of a drug into a target or non-target tissue or organ. A significant barrier may lead to desirable or undesirable compartmentalization of drugs within the body of the target organism during testing or therapy. A significant barrier is one which has sufficient importance in the drug delivery process to require it to be taken into account, and therefore a significant barrier need to be simulated experimentally.

Biological cells mimicking a significant barrier are designated "barrier cells". Biological barriers separate distinct physiological compartments, and provide selected passage of molecules. The barrier cells may provide selective passage via carrier mediated transport or hinder transport by P-glycoprotein mediated efflux. In addition, the barrier cells may form tight junctions, or *zonulae occludentes,* which are specialized membrane domains that seal the intercellular spaces in epithelial and endothelial cells and thus contribute to the permeability barrier between lumenal and interstitial compartments. The tight junctions of the barrier cells not only separate distinct physiological compartments, but they also confer selectivity to the transcellular flux of molecules and ions through the intercellular spaces between the barrier cells, the so-called paracellular pathway. In addition, tight junctions limit the diffusion of lipids between the apical and basolateral plasma membrane domains of the barrier cells. The biological cells may mimic biological barriers, such as the blood-brain barrier (BBB), the blood-testis barrier (BTB), the placental-barrier (PB), the gastro-intestinal barrier (GIB) or the skin. The barrier cells, e.g. mimicking the BBB, may provide a dynamic physical and metabolic barrier consisting of specialised cells, such as endothelial or epithelial cells. The barrier cells provide an obstruction to the passage of compounds, such as drugs. The barrier cells, such as brain capillary endothelial cells, may form tight junctions and present other barrier properties. In particular, the barrier cells may lack fenestration and pinocytotic vesicles. The barrier cells may contain metabolic enzymes and transporter proteins. For instance, the barrier cells may include transporters facilitating the uptake or impediment of entry of substrates. The barrier may comprise specialised influx transporters, such as the glucose transporter, and/or efflux transporters, such as the multidrug transporter P-glycoprotein. The barrier cells may form a barrier mimicking the blood-testis barrier (BTB), i.e. mimicking the membrane in the testis that separates sperm from the bloodstream. As such, the barrier may form specialized nonfenestrated tightly-joined endothelial cells, mimicking a transport barrier for certain substances between the testis capillaries and the seminiferous epithelium. The barrier cells may provide tight junctions between Sertoli cells. In addition, the barrier may mimic the placental barrier, i.e. the semi permeable layer of foetal tissue separating the maternal from the foetal blood in the placenta acting as a selective membrane regulating passage of substances from the maternal to the foetal blood. The barrier mimicking the PB comprises in particular syncitiotrophoblasts, cytotrophoblasts, villi connective tissue, and/or foetal capillary endothelium. The barrier mimicking the GIB comprises in particular cells which differentiate in columnar epithelium comprising tight junctions, and can be used as an *in vitro* model for the prediction of drug absorption across the human intestine.

The barrier cells may provide a monolayer of cells. In particular, the cells may be contact-inhibited in that they will not grow on top of or over each other. As such, the barrier provided by the barrier cells will be one cell wide. In the alternative, the barrier may be multilayered, e.g. cells from different origin may be located on top of each other, such as for instance mimicking the PB, or the cells forming the barrier layer are not contact inhibited and will grow over each other. Accordingly, the present invention relates to a device as described herein, wherein said layer of barrier cells is at least a monolayer of at least one cell type. Also, the present invention relates to a device as described herein, wherein said layer of barrier cells adheres to and covers, and forms for instance a continuous layer on entirely said region, and possibly entirely said second surface of said porous support (B).

In the context of the present invention, the term "tight junctions" relates to two or more closely apposed cytoplasmic membranes of adjacent cells without an intervening gap. In general, the tight junctions seal adjacent cells in a narrow band just beneath their apical surface. The tight junctions may prevent the passage of molecules and ions through the space between cells. So materials must actually enter the cells (by diffusion or active transport) in order to pass through the tissue. Thus the tight junctions provide control over what substances are allowed through. In addition, the tight junctions may block the movement of integral membrane proteins between the apical and basolateral surfaces of the cell. Thus, the barrier cells forming tight junctions may preserve the special functions of each surface of a biological barrier, such as receptor mediated endocytosis at the apical surface and exocytosis at the basolateral surface. In this regard, the drugs are placed on the apical side of the barrier cells, e.g. on support (A) and their passage through the barrier layer to the basolateral side of the cells is determined.

The barrier cells may comprise tight junction-associated integral membrane proteins. To date, these include JAM (junctional-associated molecule), a type I membrane protein of the immunoglobulin gene super family, and two types of four-transmembrane domain proteins: occludin and the claudins. JAM seems to be capable of homotypic interaction via its extracellular domain, thus producing cell-to-cell adhesion, and was implicated in the process of monocyte infiltration through an endothelial monolayer. Occludin and the members of the claudin family exhibit two short hydrophobic extracellular loops that are potentially involved in homotypic or heterotypic interactions between adjacent cells and hence in both permeability barrier and selectivity functions.

Any barrier cell capable of forming a significant barrier may be used in the device of the present invention. Accordingly, the present invention relates to a device as described herein, wherein said barrier cells are eukaryotic cells, preferably eukaryotic tissue culture cells, preferably endothelial cells capable of forming tight junctions or other closely packed cell types that present a significant barrier to the penetration of drugs.

The Caco-2 cell line, derived from a human colorectal carcinoma, provides an *in vitro* model for the prediction of drug absorption across the human intestine. When cultured on semi permeable membranes, Caco-2 cells differentiate into a highly functionalised epithelial barrier with remarkable morphological and biochemical similarity to the small intestinal columnar epithelium. The membrane transport properties of drugs can thereby be assessed using these differentiated cell monolayers. Mandin Darby Canine Kidney (MDCK) cells also provide a model for studying drug transport mechanisms in distal renal epithelia. Like Caco-2 cells, MDCK cells differentiate into columnar epithelium and form tight junctions when cultured on semi-permeable membranes. Primarily for passively absorbed compounds, drug permeability data from MDCK cell assays are similar to permeability data from Caco-2 cell assays. As a consequence, MDCK cells may provide an alternate model to Caco-2 cells for rapidly screening drugs for absorption potential, as part of the pre-clinical drug selection process. In addition, Bovine Brain Endothelial Cell (BBEC) and mouse brain capillary endothelial cell line (MBEC4) cultures can be used as an *in vitro* model to predict drug permeability across the blood-brain barrier (BBB). Accordingly, the present invention relates to a device as described herein, wherein said barrier cells are endothelial or epithelial cells capable of forming tight junctions. Preferably, the barrier cells are chosen from the group consisting of Caco-2, MDCK, MBEC4 and BBEC cells.

The cells must be present in a sufficient number to constitute an effective significant barrier for the drugs to be tested. Accordingly, the present invention relates to a device as described herein, comprising at least about 1 to 10⁵ barrier cells, preferably about 10 to 10⁴ barrier cells, or preferably about 10² to 10³ barrier cells per region or per area.

In a further embodiment, the present invention relates to a device as described herein, wherein said barrier cells and said target cells are identical.

A further advantage of the device is *inter alia,* that the effect of chemical interactions (e.g. hormonal) between different barrier and target cells types on drug action can be assessed.

In an even further embodiment, microbial cells may form a significant barrier to the penetration of drugs. Accordingly, the present invention relates also to a device as described herein, wherein said barrier cells are microbial cells. Preferably, said microbial cells form a bio film layer. The bio film layer may be constituted of one species of microbial cells or may be constituted of more than one species of microbial cells. Accordingly, the present invention relates to a device as described herein, wherein said bio film layer comprises at least one species. The number of microbial cells forming a significant barrier is usually larger than the corresponding number of eukaryotic cells. In general, the device according to the invention may comprise at least about 10³ to 10¹¹, preferably about 10⁴ to 10¹⁰, or preferably about 10⁴ to 10⁹ microbial cells per region.

### supports

As understood within the present specification, the term "first and second surfaces" of a support refers to the outer opposite sides of a support. For a porous support, said first and second surfaces may therefore be physically distinct surfaces interconnected by an intermediate material having a plurality of through-going pores or channels or may be an integral part of a porous material. The first and second surfaces often correspond to the upper and lower surfaces, respectively. Also for a non-porous support or a solid support, the first and second surfaces may be physically distinct surfaces interconnected by an intermediate material.

A number of materials suitable for use as support in the present invention have been described in the art. Materials particularly suitable for use as support (B) and/or (A) in the present invention include any type of porous support known in the art and in particular solid porous supports. The term "porous support" as used in the present specification refers to a support possessing or full of pores, wherein the term "pore" refers to a minute opening or micro channel by which matter may be either absorbed or passed through. Particularly, where the pores allow passing-through of matter, the support is likely to be permeable.
According to an embodiment, the device as described herein comprises a support (A), which is a porous support. In an alternative embodiment, the device as described herein comprises a support (A), which is a non-porous support.

A suitable support pore diameter ranges from 150 to 250 nm, including 160, 170, 180, 190, 200, 210, 220, 230 and 240 nm. A particular suitable example of pore diameter is 200 nm. These dimensions are not to be construed as limiting the present invention, however.

It is understood that porous supports according to the present invention may be semi-porous. Semi-porous supports can be induced to become fully porous by e.g. a chemical treatment or an illumination treatment. The use of semi-porous supports is advantageous in particular if the mixing of (short living) components within a supply chamber compartment(s) and/or within the pores of the porous support in a synchronous manner at a certain time in an assay is envisaged or required.

The porous support may be in the form of porous sheets, films or membranes. For example, the support may consist of fibres (such as glass wool or other glass or plastic fibres), glass or plastic capillary tubes, or metal oxide membranes. The porous support may have a simple or complex shape. The surface to which the barrier and/or target cells are adhered may be an external surface or an internal surface of the porous support. Particularly where the support material is porous, the target cell, the fraction thereof or the component thereof is likely to be attached to an internal surface, depending on the size of the target cell and the pore. Where the support is porous, various pore sizes may be employed depending upon the nature of the system. According to an embodiment of the present invention, devices are provided wherein said porous support (B) and/or said porous support (A) is a flow-through support.

The material of the porous support may be, for example, a metal, a ceramic metal oxide or an organic polymer. As a metal, for example, a porous support of stainless steel (sintered metal) may be used. Above all, a metal oxide may be used. Metal oxides provide a support having both a high channel density and a high porosity, allowing high density arrays comprising different target cells per unit of the surface for sample application. In addition, metal oxides are highly transparent for visible light. Metal oxides supports are relatively cheap that do not require the use of any typical micro fabrication technology and, that offer an improved control over the liquid distribution over the surface of the support, such as electrochemically manufactured metal oxide membrane. Metal oxide membranes having through-going, oriented channels may be manufactured through electrochemical etching of a metal sheet. Accordingly, the present invention relates to a device as described herein, wherein said porous support (A) and/or said porous support (B) is a metal oxide support.

The kind of metal oxide is not especially limited. Metal oxides considered are, among others, oxides of zirconium, mullite, cordierite, titanium, zeolite or zeolite analogue, tantalum, and aluminium, as well as alloys of two or more metal oxides and doped metal oxides and alloys containing metal oxides. Accordingly, in a further embodiment of the present invention, a device is provided, wherein said metal oxide support is an aluminium oxide support.

Metal oxide supports or membranes as employed in the methods of the present invention may be anodic oxide films. As well known in the art, aluminium metal may be anodised in an electrolyte to produce an anodic oxide film. The anodisation process results in a system of larger pores extending from one face and interconnects with a system of smaller pores extending in from the other face. Pore size is determined by the minimum diameters of the smaller pores, while flow rates are determined largely by the length of the smaller pores, which can be made very short. Accordingly, such membranes may have oriented through-going partially branched channels with well-controlled diameter and useful chemical surface properties. Useful thicknesses of the metal oxide supports or membranes as employed in the methods and apparatuses of the present invention may for instance range from 50 µm to 150 µm (including thicknesses of 60, 70, 80, 90, 100, 110, 120, 130 and 140 µm). A particular suitable example of support thickness is 60 µm.

Due to the characteristic porous structure of the supports according to the present invention, minimal amounts of target cells may be deposited on its outer surface.

Advantageously, metal oxide membranes as described herein are transparent, especially if wet, which allows for assays using various optical techniques. WO 99/02266 which discloses the Anopore^{™} porous membrane or support is exemplary in this respect, and is specifically incorporated by reference in the present invention.

Particularly useful supports employed as support (A) in the devices and methods described in the present specification are 3-dimensional porous supports, which allow pressurized movement of fluid, e.g. the sample solution, through its structure. These porous supports as employed in the present devices and methods possess a permeable or flow-through nature. In contrast with two-dimensional supports, 3-dimensional supports or micro arrays as employed in the devices and methods as described herein give significantly decreased release times, when a positive pressure is applied to the support in order to pump the effector molecule or drug dynamically through the support pores with a tight control of the volume pumped. Said dynamical pumping allows immediate removal and ability to perform real-time detection of efficacy from a passage through the barrier cells. Flow-through supports are particularly suitable if the relation between the spatial position of a compound within the porous solid support and the signal is to be retained.

The nature and geometry of the solid porous support as useful in the present invention will depend upon a variety of factors, including, among others, the type of array and the mode of attachment (e.g., covalent or non-covalent). Generally, the porous support (B) according to the present invention may be composed of any porous material which will permit adherence of barrier cells and which will allow potential passage of drugs. The support (A) according to the present invention may be composed of any material, including porous material, which will permit attachment and controlled release of drugs.

In this regard, the porous support (B) is used as basis support for the growth and/or adherence of barrier cells. Generally, barrier cells will be seeded on the porous support (B), after which the barrier cells, attach to the porous support (B) and possibly grow, divide, differentiate and/or migrate. The barrier cells may contact each other and form a monolayer. Where barrier cells contact each other, tight junctions may be formed. In order to enhance biocompatibility of the support and cell adhesion, the support may be coated. Exemplary coatings are for instance, poly-L-lysine, gelatine and Matrigel (Becton-Dickinson), and other basement membrane components, such as collagens, laminin, entactin, and/or proteoglycans. In addition, the coating may contain growth factors enhancing the growth, division, differentiation and/or migration of the barrier cells. Accordingly, the present invention relates to a device as described herein, wherein said support (A) and/or porous support (B) is coated for enhancing biocompatibility, cell adhesion and/or other cellular properties, including growth.

### media

In order to maintain the viability of the target cells and/or the barrier cells, the device and methods as described herein may comprise a culture medium with sufficient amounts of oxygen and nutrients. In particular, the culture medium composition may comprise basic nutrients (sugars, amino acids) and growth factors/cytokines. It will be understood that the passage of particular sugars, amino acids and growth factors/cytokines through the barrier cells can be investigated by depletion of these sugars, amino acids and/or growth factors/cytokines in the culture medium sustaining the target cells, while said particular sugars, amino acids and growth factors/cytokines are present in the medium sustaining the barrier cells. Accordingly, the present invention relates to a device as described herein, further comprising culture medium on the second surface and possibly the first surface of the porous support (B), which may sustain the growth of the barrier cells and/or target cells.

The pH, serum content or other aspects of the medium may differ for the two sides of the porous support (B) to cater to the barrier and target cells needs. Accordingly, the present invention relates to a device as described herein, wherein said culture medium on the second surface and on the first surface of the porous support (B) are different.

In order to further control the culture medium, the porous support (B) may comprise intermediate molecules that do not enter the solution. These intermediate molecules are for instance charged polymers that affect the pH of the environment. Accordingly, the present invention relates to a device as described herein, wherein said porous support (B) further comprises intermediate molecules that do not enter into solution.

Obviously, the intermediate molecules may alter the properties of the drugs under investigation. For instance, a local change in pH may either activate or inactivate a drug. These properties may be investigated. Consequently, the intermediate molecules may provide a further level of complexity to testing the activity of drugs. Hence, an advantage of the device and method is *inter alia,* the local manipulation of the drug/cellular environment within the pores of the porous support (A) and/or (B), e.g. on the pH, allowing the passage of a drug to be tested under a range of different conditions within the same array format. Accordingly, the present invention relates to a device as described herein, wherein said intermediate molecules are arrayed, said arraying may be done by means of any method known in the art.

### drugs and drugs release

The devices and methods according to the invention are particularly suited for testing the efficacy of drugs passing the barrier layer, providing a significant barrier, and possibly mimicking a biological barrier. The device is useable for determining the pharmacokinetics of the drugs under investigation, such as determining the fate of the drugs from the time they are introduced into the body until they are eliminated, and especially the capability of passing a significant barrier, such as a biological barrier. The device according to the invention may contain a mechanism by which the test compounds, i.e. the drugs, can be introduced to the system. In the present invention, the term "drug" relates to any animal, vegetable, or mineral substance used in the composition of narcotics or medicines, i.e. a compound used to treat disease, injury or pain.

The methods and devices according to the present invention allow the use of a large diversity of drug libraries including biologically active drug libraries as well as drug libraries without disclosed biologically activity. Typical biologically active drug libraries are the so-called discovery drug libraries, targeted drug libraries and optimisation drug libraries. The term "discovery drug library" refers to large size (>5000) members and has no preconceived notion about which molecular target(s) or target cell(s) the drugs may be active against. The "targeted drug library" is biased in its design and refers to a drug library that contains a pharmacophore known to interact with a specific molecular target, family of molecular targets or target cell. The term "optimisation drug library" refers to a drug library in which a lead exists and an attempt is being made to improve, for example, its potency, selectivity, pharmacokinetic profile or efficacy.

General suitable classes of compounds for use in the methods and devices according to the present invention include, by way of example and not limitation, natural drugs derived e.g. from plants with defined therapeutic applications, chemically synthesized drugs, drugs derived from combinatorial chemistry, peptide-based drugs, peptide derivatives and the like. Accordingly, the present invention relates to a device as described herein, wherein said drugs of said solid support (A) are compound libraries. The term "compound libraries" refers to a library of compounds, in which said compounds may be derived from any source, such as, for instance, compounds derived from combinatorial chemistry, compounds derived from natural sources, such as biologically active drugs, compounds derived from peptide chemistry, compounds derived from chemical synthesis, and the like, as will be apparent to the person skilled in the art. Biologically active drug libraries may include proteolytic enzymes, such as, for example, serine proteases like trypsin, non-proteolytic enzymes including inducer molecules, chaperone proteins, antibodies and antibody fragments, agonists, antagonists, inhibitors, G-coupled protein receptors (GPCRs), non-GPCRs, and cytotoxic and anti-infective agents. Examples of drug libraries without disclosed biologically activity may include scaffold derivatizations, acyclic synthesis, monocyclic synthesis, bicyclic and spirocyclic synthesis, and poly and macrocyclic synthesis of drugs, or drugs which interact with any of the above-mentioned molecules. Accordingly, the present invention relates to a device as described herein, wherein said drugs are chosen from the group consisting of chemical compounds, natural compounds, oligo-peptide-based compounds, derivative peptide compounds, biologically active compounds, potential drug candidate compounds, and compounds potentially able to modulate cell growth, differentiation, apoptosis and/or migration. Compounds obtained through combinatorial and so-called fast synthesis may be equally suitable. In a further embodiment, said compounds are drugs selected from a chemical or natural drug candidate library. Accordingly, the present invention relates to a device as described herein, wherein said compounds are drugs selected from a chemical or natural drug candidate library.

In a particular embodiment of the present invention, devices and methods are provided wherein said drugs are chosen from the group consisting of enzymes, enzyme substrates, inducer molecules, enhancer molecules, inhibitor molecules, chaperone proteins, transcription factors, differentiation-inducing or inhibiting agents, migration-inducing or inhibiting agents, apoptotic inducing or inhibiting agents, growth inducing or inhibiting agents, secondary metabolites, toxins, glycolipids, carbohydrates, antibiotics, mutagens, hormones, oligopeptides, nucleic acids, agonists, antagonists, ligands, aptamers, monoclonal and polyclonal antibodies, synthetic variants of nucleic acids such as PNAs or LNAs, inhibitors of cellular functions, enhancers of cellular functions, anti-viral agents, anti-fungal agents, growth factors, pharmaceutical compositions, vaccine preparations, cytotoxic chemicals, cytokines, chemokines, chemotherapeutic agents, macromolecular structures, such as viruses modified for the delivery of therapeutic agents (e.g. in gene therapy), nano-molecular delivery particles, levodopa, dopamine agonists and/or any combination thereof. Said monoclonal antibodies may be engineered monoclonal antibodies or may be therapeutic monoclonal antibodies. These drugs are particularly suitable for use within the present invention.

Drugs which may be tested with the devices and methods of the invention comprise for instance centrally acting drugs (anti-dementia drug, therapeutic agents for brain tumours, viral therapeutic agents, and drugs acting on nerves), drugs targeting various receptors expressed in brain capillary endothelial cells (brain microcirculation improving drugs, therapeutic drugs for brain oedema) drugs targeting cytopathy (brain angiopathic dementia, Alzheimer's dementia) of brain capillary endothelial cells. The present invention relates particularly to devices and methods described herein, wherein said drug affects CNS-diseases such as Parkinson's disease, Alzheimer's disease, psychiatric conditions, such as depression or Schizophrenia, or infections such as bacterial, fungal, viral meningitis or prion disease.

It will be understood that the drugs of the invention are assessed of passing the barrier layer, in case the drugs are supposed to pass the barrier layer, and in case the drugs are not supposed to pass the barrier layer. In a further embodiment of the present invention, devices and methods are provided wherein the drugs are chosen from the group of compounds having neuro-activity. Thus, the effect of the BBB is assessed on the delivery of some drugs to the CNS. In addition, the present invention relates to devices and methods whereby the drugs are chosen from the group of compounds which are not supposed to go to the CNS, i.e. wrongly delivered to the CNS causing undesirable side-effects, such as drowsiness. Also, the present invention relates to devices and methods whereby the drugs are chosen from the group of compounds which are being assessed for penetrating the PB, e.g. having an effect on the embryo. In general, drugs are being tested for absorption via the GIB.

Attachment of the drugs on and/or in support (A) can be done by any means known to the person skilled in the art, for instance, the drugs may be printed on support (A). The drugs may be covalently or non-covalently attached to the support (A), provided the drugs can be released from the support. As such, the drugs may be immobilised to the support (A) by adsorptive attachment. Accordingly, the present invention relates to devices and methods as described herein, wherein said drug is reversibly immobilized by covalent attachment or adsorptive attachment to, in and/or on said support (A). Also, the drugs may be attached to support (A) by non-covalent interactions such as hydrophobic or ionic interactions. Delivery of the drugs to predefined regions and/or areas on the apical site of the porous support (B) may also be accomplished by using a liquid handling device, such as printing, but may equally be accomplished by manual handling, e.g. using a pipettor. Accordingly, the drugs need not be present on support (A). It will further be appreciated that the drugs can be arrayed at predefined regions and/or areas on and/or in support (A), preferably in rows and columns, by means of any method known in the art.

The drugs may be maintenance-free and quality controlled stored prior to testing. Drugs may be stored on the support after a drying treatment, after which they can be dissolved again, later on when an assay needs to be performed. The drugs may be dried on the support, possibly with an agent that controls their release upon re-hydration. Removal by drying of the solvent normally used to dispense the drugs provides the additional advantage of avoiding possible interference of said solvent with the subsequent assay. Accordingly, the present invention relates to a device as described herein, wherein said drugs are stored in dried or lyophilised condition in and/or on said support (A).

Upon drug dissolution; e.g. when in contact with an appropriate liquid or buffer, the drugs diffuse from the support and contact the barrier layer. Depending on the solubility of the drug, diffusion may be total or partial and sufficient to allow for target identification. Accordingly, the present invention relates to a device and method as described herein, wherein said drugs are released by passive diffusion. In order to regulate the release of the drug by passive diffusion, the present invention contemplates a device as described herein, wherein said drugs are coated, preferably with matrigel, poly-L-lysine, gelatine or carageenin. The amount of the coating may be adjusted by the person skilled in the art, dependent upon the need, e.g. when testing pharmacokinetic properties of drugs. Drugs can also for instance printed as 300 micron diameter spots in an ion exchange polymer such as polyurethane or acrylic resins to slow the drug release upon re-hydration from hours to days.

Transfer of the drugs is not limited to diffusion, and may also be by providing a pressure difference over support (A), wherein the pressure at the support (A) is higher than the pressure at the solid porous support (B), thereby maximising the release of the drugs, delivering the drugs by micro fluidic channels. Accordingly, the present invention relates to a method and device as described herein, wherein said drugs are released by providing a pressure difference over support (A), wherein the pressure at the support (A) is higher than the pressure at porous support (B). The present invention further contemplates that the drugs are released by heat, electromagnetic radiation, e.g. light, or biological mechanisms, such as for instance the action of enzymes. Mechanisms to control the release of drugs may further be effected by hydrophobic or ionic interactions between said drug and the coating on the support.

In case of a porous support (A), the drugs may be retained within the pores and may present a high concentration of drug to the barrier cells despite small amounts of material being attached to, e.g. printed on a porous support (A), as drugs can accumulate within the small-volume pore localized in close proximity of the barrier cells.

In order to facilitate tracking of the drugs through the barrier layer, and possibly the subsequent binding to the target cells, these drugs may be tagged. A variety of different labels may be employed, where such labels include fluorescent labels, isotopic labels, enzymatic labels, particulate labels, etc. For example, suitable labels include fluorochromes, e.g. fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxy-fluorescein (JOE), 6-carboxy-X-rhodamine (ROX), 6-carboxy-2',4',T_{,}4,7-hexachloro-fluorescein (HEX), 5-carboxyfluorescein (5-FAM) or N,N,N',N'-tetramethyl-6-carboxy-rhodamine (TAMRA), cyanine dyes, e.g. Cy5, Cy3, BODIPY dyes, e.g. BODIPY 630/650, Alexa542, etc. Suitable isotopic labels include radioactive labels, e.g. ³²P, ³³P, ³⁵S, ³H. Other suitable labels include size particles that possess light scattering, fluorescent properties or contain entrapped multiple fluorophores. The label may be a two stage system, where the drug is conjugated to biotin, haptens, etc. having a high affinity binding partner, e.g. avidin, specific antibodies, etc. The binding partner is conjugated to a detectable label, e.g. an enzymatic label capable of converting a substrate to a chromogenic product, a fluorescent label, an isotopic label, etc. It will be understood that in case a drug is labelled, the label chosen will preferably not interfere with the determination of the drug efficacy, i.e. the passage of the drugs through the significant barrier. Accordingly, the present invention relates to a method and device as described herein, wherein said tag is a labelled marker. Preferably, said labelled marker is a fluorescent marker.

In addition, to the drugs, other compounds (such as charged polymers that do not enter the solution but affect the pH of the solution) may be attached together with the drugs to the support (A) to locally manipulate the environment of the drugs as it contacts the barrier cells.

### methods for making the device

The porous support (B) can be made by placing the support in a container comprising culture medium an seeding the barrier cells, under conditions allowing these cells to adhere to the porous support (B) and the growth, division and/or differentiation thereon, until a barrier layer is formed presenting a significant barrier, e.g. comprising tight junctions, and mimicking a biological layer. Accordingly, the present invention relates to a method for making the device as described herein, comprising seeding barrier cells on the second surface of said porous support (B) under conditions allowing the adherence to said second surface and growth of said barrier cells, whereby said barrier cells form tight junctions or other connections presenting a significant barrier.

Similarly, the support (A) and the support (W) can be made by placing the support (A) or (W) in a container comprising culture medium an seeding the target cells, under conditions allowing these cells to adhere to the support (A) and the support (W), respectively, and the sustainment, growth, differentiation and/or division thereon. It will be appreciated that said target cells can be maintained in the compartments of the support (A) or (W) when present.

The device according to the invention may, in addition to the barrier cells on the second surface of the porous support (B), further comprise the target cells adhered to the first surface of the porous support (B). Accordingly, the present invention relates to a method for making the device as described herein, further comprising seeding target cells on the first surface of said porous support (B) under conditions sustaining said target cells. In general, first the barrier cells will be seeded on the second surface of said porous support (B) under conditions allowing the adherence to said second surface and growth of said barrier cells, whereby said barrier cells form a significant barrier, such as for instance, tight junctions, after which the support is inverted and the target cells are seeded on the first surface of the porous support (B), under sustaining conditions of the barrier cells as well as the target cells.

In case the porous support (B) and the supports (A) and/or (W) are preformed, i.e. comprise cells adhered thereto, the supports can be assembled in a housing. As such, the present invention relates to a method for making the device as described herein, comprising sandwiching the layer of barrier cells on said second surface of the porous support (B) between said second surface of the porous support (B) and support (A).

In an alternative embodiment, the present invention relates to a method for making the device, wherein said barrier cells are seeded between the second surface of said porous support (B) and said support (A). The cells are seeded under conditions allowing these cells to adhere to the porous support (B) and possibly support (A) and allowing the growth, division and/or differentiation thereon, until a significant barrier, such as for instance a barrier layer is formed comprising tight junctions, and mimicking a biological layer.

In case the barrier cells are microbial cells, possibly forming a bio film, the significant barrier will generally preformed on porous support (B), i.e. the microbial will be seeded, grown and sustained on porous support (B). Condition for seeding, growing and sustaining microbial cells are well known in the art. This preformed support may be stored until use under preserving conditions, such as at 4°C. When a drug or drug library is to be tested, said drug(s) are contacted with the preformed support, the efficacy of drugs to pass the significant barrier, e.g. a microbial bio film, is determined.

### methods and uses

The device according to the invention may be used in methods for determining drug efficacy on target cells, components or fractions of target cells. The device may also be used in methods for determining biological activity, toxicity, side-effects or teratogenicity of drugs on target cells, components or fractions of target cells. The device is particular useful in methods for determining the efficacy of drugs for passing barrier cells. Also, the device may be used for determining efficacy of drugs for passing biological barriers, such as blood-brain barriers, blood-testis barriers, gastro-intestinal barriers and/or placenta barriers. In addition, it is convenient to use the device in methods for determining the efficacy of drugs for passing a layer of cells comprising tight junctions. In this regard, the device would be useful in methods for determining efficacy of drugs for passing a layer of brain capillary endothelial cells or epithelial cells. Notably, the device is preferably used in methods for determining efficacy of drugs for overcoming P-glycoprotein mediated efflux, e.g. in brain capillary endothelial cells.

The term "efficacy" relates to the ability and in particular the effectiveness of a drug to pass the significant barrier, such as a layer of barrier cells, possibly via the tight junctions. Efficacy should be distinguished from activity, which is limited to a drug's immediate effects on target cells, components or fractions of target cells.

An advantage of the method is *inter alia,* that passage assays can be highly multiplexed, and can be combined with an ultimate cellular outcome of a drug (e.g. toxicity, activity) within a single array format.

The present invention relates in particular to a method for determining drug efficacy on target cells comprising:
(i) providing a device as described herein, i.e., a porous support (B) having first and second surfaces and at least one area with a plurality of through-going channels, wherein said area comprises on said first surface at least one region, which can be contacted with target cells, and said area comprises on said second surface at least one region comprising a layer of barrier cells presenting a significant barrier adhered to said second surface, wherein said region on the first surface is located opposite to said region on the second surface of the porous support (B), and wherein said layer of barrier cells provides a barrier for the passage of drugs;
(ii) contacting said at least one region on said first surface of said porous support (B) with target cells;
(iii) contacting said layer of barrier cells on the second surface of the porous support (B) of said device with a support (A) comprising drugs;
(iv) incubating said layer of barrier cells with said drugs under conditions allowing the passage of said drugs through porous support (B), and allowing binding of said drugs to target cells; and,
(v) determining whether the drugs have bound to the target cells, whereby drug efficacy is determined, such as the passage of said drugs through significant barrier, e.g. through the junctions of said barrier cells or through the cells themselves.

In a further embodiment, the said target cells are on the surface of a support (W) which is contacted with the first surface of the porous support (B). Accordingly, the present invention relates also to a method for determining drug efficacy on target cells as described herein, comprising:
(i) providing a device as described herein
(ii) contacting target cells on the surface of the support (W) with the first surface of said porous support (B), wherein the layer of barrier cells adhered to said second surface provides a barrier for the passage of drugs;
(iii) contacting the layer barrier cells on the second surface of the porous support (B) of the device with drugs,
(iv) incubating the layer of barrier cells with said drugs under conditions allowing passage of said drugs through porous support (B), and allowing binding of said drugs by said target cells, and
(v) determining whether the drugs have bound to the target cells, whereby drug efficacy is determined, such as by determining the passage of said drugs through the significant barrier, e.g. through the junctions of said barrier cells or through the cells themselves.

Considering that said drugs may be comprised in and/or on support (A), it will be appreciated that the present invention also relates to a method for determining drug efficacy on target cells, the fractions or components thereof, as described herein, wherein said drugs are comprised in and/or on support (A) of the device, and said incubating is further under conditions allowing the release of the drugs from said support (A).

It will be appreciated that monitoring the drugs through their passage of the significant barrier, e.g. through the barrier cells may be also useful. Hence, the present invention relates in a further aspect to determining whether the drugs are present in and/or on the barrier cells, in addition to or in the alternative of determining whether the drugs have bound to the target cells, the fractions or components thereof. As a further advantage of the present invention, the effect of metabolism of drugs by the barrier cells can be assessed on the target cells, the fractions or components thereof (e.g. metabolism of a pro-drug to drug during passage).

### determination

Determining, such as monitoring or analysing the interaction, between the drug and the target cell can be performed by any means known in the art. In general the interaction between the drug and the target cell, the fractions or components thereof, will be a binding, resulting in a detectable signal. The signals generated by the interaction may be transient, i.e. passing especially quickly into and out of existence. The term "transient signal" as used in the present invention refers to e.g. a pulse. The level and intensity of said pulse may be directly linked to the interaction. In addition, the efficacy of passing the barrier layer may be assessed by determining the concentration of the drug on the basolateral side of the barrier cells relative to the concentration of the drug on the apical side of the barrier cells. Provided the drug can be detected as such, the passage of the drug through the barrier layer may be determined. Accordingly, the present invention relates to a method as described herein, wherein said determining is by measuring transient changes in said target cells, the components or fractions thereof.

The signals generated may include radiation in the visible, ultraviolet, infrared and/or other portions of the electromagnetic radiation spectrum, or a difference in magnetism, charge, mass, affinity, enzymatic activity, or reactivity. It will be understood that the interaction analysed according to the present invention may be monitored via, e.g. chemiluminescence, bioluminescence, phosphorescence, fluorescence or radioactive radiation. Detection methods of the generated signals are well known in the art. Signals may be detected or visualized in a variety of ways, with the particular manner of detection being chosen is based on the reporter system which is utilized. Representative detection means include scintillation counting, auto-radiography, optical detection such as fluorescence measurement, colorimetric measurement, light scattering, and the like. Accordingly, the present invention relates to a method as described herein, wherein said determining is by a method chosen from the group comprising luminescence microscopy, regular light microscopy, confocal microscopy, electron microscopy, UVNIS absorbance, microcalorimetry, laser flow-cytometry, radiometry and/or phosphoimaging. Preferably, said luminescence is chosen from the group consisting of fluorescence, time-resolved fluorescence, lifetime fluorescence, or electrochemiluminescence.

The interaction can be screened using a binding assay or a functional assay that measures downstream signalling, such as measuring changes in second messenger pathways, e.g. through a receptor. The functional assays can be performed in e.g. isolated target cell membrane fractions or on target cells expressing the receptor interacting with the drug on their surfaces. Alternatively, the drug may affect signalling downstream from a receptor without interaction of said drug with said receptor. Any of the assays of receptor activity, including but not limited to the GTP-binding, GTPase, adenylate cyclase, cAMP, phospholipid-breakdown, diacylglycerol, inositol triphosphate, arachidonic acid release, PKC, kinase and transcriptional reporter assays, can be used to determine the presence of a drug passing the barrier layer. Other functional assays include, for example, microphysiometer or biosensor assays (see Hafner, 2000, Biosens. Bioelectron. 15: 149-158, incorporated herein by reference). The intracellular level of arachinoid acid can also be determined as described in Gijon et al., 2000, J. Biol. Chem., 275:20146-20156. Accordingly, the present invention relates to a method as described herein, wherein said determining is by measuring changes in second messenger pathways. The changes effectuated by the drug in the target cell may also be determined, as well as the effects of these changes, such as cell motility, morphology, cell death, apoptosis, cell division. Accordingly, the present invention relates to a method as described herein, wherein said determining is by measuring changes in said target cells. In particular, the present invention relates to measuring changes in the level or amount of pH, oxygen, specific metabolites such as glucose or the presence or absence of an indicator molecule, expression levels of indicator genes or proteins. Also, the changes effectuated by the drug may eventually result in an effector molecule being secreted into the medium of the target cells. This effector molecule would then be indicative of the drug passing the barrier layer and interacting with the target cell. Accordingly, the present invention relates to a method as described herein, wherein said determining is by measuring changes in the medium of said target cells. In this regard, the term "measuring changes" relates to a difference, i.e. increase or decrease, of at least 10% or more, e.g. 20% or 50%, in the level or amount of the parameter under investigation, e.g. the drug, in a test relative to test without the drug, said change being indicative of the drug effectively passing the barrier layer. It will be understood that the presence of the drug as such may be measured directly when it has passed the barrier layer, i.e. without the use of binding partners, for instance in the medium at the basolateral side of the cells forming the barrier layer. As such, permeability coefficient values can be measured and expressed in nm per second. In some assays, it may be necessary to harvest the cells in order to facilitate further measuring, e.g. by scintillation counting. Accordingly, the present invention relates to a method as described herein, further comprising the step of harvesting the target cells.

The present invention particularly relates to a method for determining changes in target cells which are preloaded with a (fluorescent) dye, and changes in metabolic parameters can be assessed by measuring the change in NDD(P)H levels via intrinsic fluorescence of these molecules. Target cells can also be preloaded with a dye which leaks in the case of membrane damage, resulting in a decreased fluorescent intensity. In addition, or in the alternative, reporter genes can be transfected into the target cells under the control of, e.g. a stress-related, promoter which is activated by the drug to produce a fluorescent product.

The present invention relates particularly to ligand binding assays, in which cells expressing a particular receptor, fractions of said cells comprising said receptor, or the receptor as such are exposed to a labelled ligand and the drug. Binding of the ligand and/or the drug or displacement of binding of the ligand can be monitored by surface plasmon resonance (SPR), including the use of a Biacore Biosensor (Biacore AB). Another method of detecting drug efficacy uses fluorescence resonance energy transfer (FRET). A variation on FRET uses fluorescence quenching to monitor molecular interactions. Fluorescence polarization measurement may be useful to quantitate binding. Another alternative for monitoring drug interaction on receptor:ligand complexes uses a biosensor assay. All the methods mentioned above are described in WO 03/057730, which is incorporated herein by its entirety.

It is important to note that in assays testing drugs on the interaction of ligands with receptors, it is possible that a drug affecting the interaction need not necessarily interact directly with the domain(s) of the receptors that physically interact with the ligand. It is also possible that a drug will interact at a location removed from the site of interaction and cause, for example, a conformational change in the receptor. Drugs, such as inhibitors or agonists, that act in this manner are nonetheless of interest as agents to modulate the activity of receptors.

The present invention relates particularly to functional assays, in which a signalling activity of a receptor is measured. Target cells expressing a particular receptor, fractions of said cells comprising said receptor, or the receptor as such are incubated with a drug, and the signalling activity of the receptor is measured. The activity induced by the drug that modulates receptor activity may be compared to that induced by its natural ligand, whereby the efficacy of drugs passing the barrier layer is determined. The following functional assays are especially contemplated by the present invention: GTPase/GTP binding assays, calcium flux (aequorin-based) assay, adenylate cyclase assay, cAMP assay, phospholipid breakdown, DAG production and Inositol triphosphate (IP₃) levels, PKC activation assays, Kinase assays, transcriptional reporters for downstream pathway activation, Inositol phosphates (IP) measurement, all as described in WO 03/057730, which is incorporated herein by its entirety.

In this regard, a ligand releases a signal by binding to and activating a protein molecule, i.e. a "receptor," which is present on the surface of another cell. Once the ligand binds to the receptor on the cell surface, the receptor undergoes a conformational change which, in turn, triggers a cascade of events that transmit the signal to the inside of the cell. This signal ultimately causes the cell to undergo some sort of "action". The specific "action" on the cell is determined by the specific "ligand" and "receptor" involved. This "action" may come in the form of a change in shape of the cell, the movement of the cell in a specific direction, the opening of channels on the cell surface that allow items to move in and' out of the cell, the transmission of an electrical impulse that travels through the cell, changes or activates gene expression, changes or activates specific second messenger pathways such as described hereinbefore, or the secretion of some chemical that may in turn bind to another cell and impose an "action" on it. Hence, a drug may be a ligand.

### kits

The present invention relates also to kits comprising the device according to the invention and possibly an instruction manual. In particular, a kit comprising a porous support (B) having first and second surfaces and at least one area with a plurality of through-going channels, a support (A) comprising drugs, and barrier cells and/or target cells is contemplated. In addition, the present invention relates to a kit comprising a solid support (W) comprising target cells, a porous support (B) having first and second surfaces and at least one area with a plurality of through-going channels, optionally comprising a layer of barrier cells adhered to said second surface, and optionally a support (A) comprising drugs.

### uses

The devices of the invention can be used in determining the efficacy of drugs passing a significant barrier, such as a biological membrane, for instance in high throughput screening. The device is a complex cellular array designed to assay drug permeability through relevant biological barriers, penetration being detected by the biological effects of the drugs on the second cells, i.e. the target cells. The method using the device allows relatively complex models of drug action to be used to monitor penetration (e.g. passage) plus efficacy or toxicity in a single, miniaturized and highly multiplexed assay. In particular, the devices of the invention can be used in screening to identify centrally acting drugs (anti-dementia drug, therapeutic agents for brain tumours, viral therapeutic agents, and drugs acting on nerves) based on the blood-brain barrier transmission mechanism and in screening to eliminate drugs having the problem of side effects in the nerve centre. Furthermore, the present invention relates to device which can be used in screening of drugs targeting various receptors expressed in brain capillary endothelial cells (brain microcirculation improving drugs, therapeutic drugs for brain oedema). Moreover, the present invention relates to a device which can be used in screening of drugs targeting cytopathy (brain angiopathic dementia, Alzheimer's dementia) of brain capillary endothelial cells. In particular, the present invention relates to a device which can be used for determining drug efficacy on target cells, components or fractions of target cells, for determining biological activity, toxicity, side-effects or teratogenicity of drugs on target cells, components or fractions of target cells, for determining efficacy of drugs for passing barrier cells, preferably for determining efficacy of drugs for passing blood-brain barriers, blood-testis barriers and/or placental barriers, for determining efficacy of drugs for passing a layer of cells comprising tight junctions, a layer of brain capillary endothelial cells or for determining efficacy of drugs for passing p-glycoprotein in brain capillary endothelial cells. As will be apparent, the device of the present invention can also be used for determining efficacy of drugs for passing closely packed cells that present a significant barrier to the penetration of drugs and for determining efficacy of drugs for passing microbial cells forming a bio film.

The present invention provides a substantially novel device and method for performing assays on biological micro-arrays. While specific examples have been provided, the above description is illustrative and not restrictive. Many variations of the invention will become apparent to those of skill in the art upon review of this specification. The scope of the invention should, therefore, be determined not with reference to the above description, but instead should be determined with reference to the appended claims.

### SHORT DESCRIPTION OF THE FIGURES

**Figure 1** Porous support (B) comprising barrier cells
   Porous support (B) (10) having opposite first (11) and second (12) surfaces, and comprising barrier cells (13) on the second surface (12) of said porous support (B)(10). The layer of barrier cells (13) presenting a significant barrier are barrier cells forming tight junctions (14). Drugs would have to travel from the apical to the basolateral side.
**Figure 2** Barrier cells sandwiched between porous support (B) and support (A)
   A layer of barrier cells (13) comprising tight junctions (14) is attached to the second surface (12) of support (B) (10) and brought into contact with support (A) (21) comprising a drug (22) at the apical side of the barrier cells (13).
Figure 3 Drug passing the significant barrier
   Similar as for Figure 2, a layer of barrier cells (13) comprising tight junctions (14) is attached to the second surface (12) of support (B) (10) and brought into contact with support (A) (21) comprising a drug (22). The drug (22) releases from support (A) (21), passes through the barrier cells (13) forming a significant barrier and porous support (B) (10), comes into contact with the target cells (16), present on the first surface (11) of support (B) (10). Support (B) (10) comprising the barrier cells (13) may be placed in a vial, chamber or container comprising growth medium.
**Figure 4** various embodiments of the device
   Figure 4A Porous support (B) (10) comprising barrier cells (13) attached to its second surface (12) and target cells (16) attached to its first surface (11), wherein said support (B) (10) has edges (15) on its borders projecting from the second surface (12) of support (B) (10). Porous support (B) (10) is placed into a chamber (30) with a bottom surface (W) (31), with the basolateral side, i.e. the first surface (11), of the porous support (B) (10) first. This chamber (30) comprises growth medium (32). A support (A) (21) comprising drugs (22) is depicted above the barrier cells (13), and may be placed onto the barrier cells (13) whereby the drugs (22) contact the barrier cells (13).
   Figure 4B Porous support (B) (10) comprising barrier cells (13) attached to its second surface (12). Support (B) (10) has edges (15) on its borders projecting from the second surface (12) of support (B) (10). Support B may contact the chamber wall. The device further contains a chamber (30) with a bottom surface (W) (31). Target cells (16) are attached to the bottom surface (W) (31) on the inside of the chamber. This chamber (30) further comprises growth medium (32). Porous support (B) (10) is placed into the chamber (30), with the basolateral side, i.e. the first surface (11), of the porous support (B) (10) first. The first surface (11) of support (B) (10) may contact the target cells (16). A support (A) (21) comprising drugs (22) is located above the barrier cells (13). Support (A) (21) may be placed onto the barrier cells (13) whereby the drugs (22) contact the barrier cells (13).
   Figure 4C Porous support (B) (10) comprising barrier cells (13) which are attached to its second surface (12). Support (B) (10) has edges (15) on its borders projecting from the second surface (12) of support (B) (10). The device further contains a chamber (30) with a bottom surface (W) (31), from which well dividers (33) protrude into the chamber (30). The wells (34) contain target cells (16), which are in suspension, e.g. the target cells are suspended in growth medium (32). Porous support (B) (10) is placed into the chamber (30), with the basolateral side, i.e. the first surface (11), of the porous support (B) (10) first. The first surface (11) of support (B) (10) may contact the target cells (16) physically, or via a fluid interface, e.g. the growth medium. Support (A) (21) comprising drugs (22) is located above the barrier cells (13). Support (A) (21) may be placed onto the barrier cells (13) whereby the drugs (22) contact the barrier cells (13).

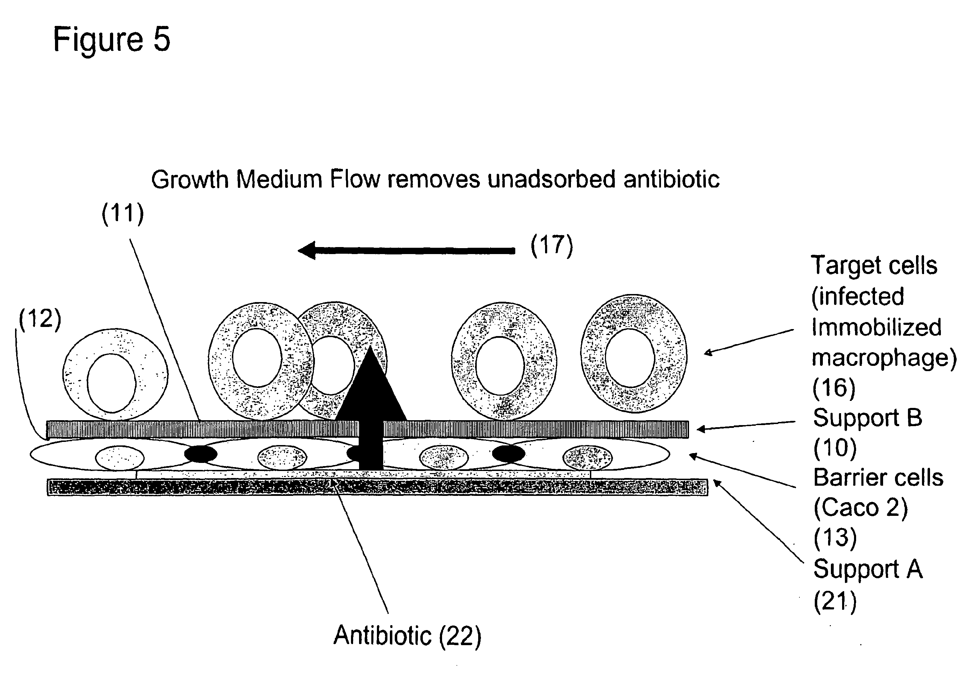

**Figure 5** Antibiotic passing significant barrier
   Porous support (B) (10) comprising barrier cells (13; Caco2 cells) attached to its second surface (12) and target cells (16; infected immobilised macrophages) attached to its first surface (11). Support (A) (21) comprising antibiotics (22) is placed at the apical side of the barrier cells (13), thereby sandwiching the barrier cells (13) between support (A) (21) and porous support (B) (10). Antibiotics (22) emanate from support (A) (21), pass the layer of barrier cells (13) and porous support (B) (10) and affects the target cells (16). Unadsorbed antibiotics are removed by growth medium flow (17).

### EXAMPLES

### Example 1 - Efficacy of Drugs Penetrating the Blood-Brain Barrier

To demonstrate an application for the invention in high content screening of drugs designed to penetrate the blood-brain barrier (BBB) and have an effect on targets within cells of the human central neural system.

A drug library of potential drugs (22) targeted against the central nervous system (CNS) is printed on the support (21) and dried. Drugs are printed as 300 micron diameter spots in an ion exchange polymer such as polyurethane or acrylic resins to slow the drug release upon re-hydration from hours to days. A 5 x 10 cm sheet of anopore is used, printed with 5,000 drugs or drug combinations to create support layer A (see figures 2 and 3). Alternatively, if the penetration of nutrients to the barrier cells (13) through layer B (10) is sufficient then layer A (21) may be non-porous.

A barrier cell layer of endothelial and glial cells (13), simulating the BBB, is formed on a second sheet of anopore support (support B) (10). Important characteristics of this layer are tight cell-cell junctions (14), active efflux transporters (e.g. Pgp), active uptake transporters, expression of drug receptors found *in vivo,* expression of drug metabolizing peptides (e.g. gamma-glutamyl transpeptidase) and similarity of responsiveness to permeability modulators as is found *in vivo.*

A second layer of target adherent cells (16) simulating a central neural system target for a drug on the reverse side of support B (10) either simultaneously or subsequently to step 2. These target cells contain multiple reporters for drug efficacy (e.g. inhibition of a specific tyrosine kinase-mediated signalling pathway indicated by expression of GFP). The target cells can be a tight monolayer or dispersed or captured onto the support.

Support layers A (21) and B (10) are brought together, as shown in Figure 3 so that the barrier cells (13) are sandwiched between them. The entire cell-penetration array is incubated in mammalian growth medium in a CO₂ incubator at 37 °C (Fig. 4). Despite forming tight monolayers both barrier (13) and target (16) cells have access to growth medium. Each drug spot will start to re-hydrate and penetrate the barrier cells (13) directly or test the junctions between cells (14). In this example, a barrier is placed below the support layer A (10) is to direct drug delivery into the barrier cells (13). The volume of medium above the target cells (16) is large, and/or slowly re-circulating to ensure drugs that are not taken up by the cells directly from the pores are dispersed.

At specific time periods (0, 2, 4, 8, 16, 32 hours) the target cells are imaged and putative drug candidates rapidly determined after image analysis and quantification of the GFP signal. Imaging is by fluorescence microscopy and data capture by CCD camera. Computational analysis using imaging software is used to flag the locations of events that indicate lead drug candidates and identify errors or unusual events (e.g. synergistic or antagonistic action of drugs in adjacent spots).

At 32 hours+ cells are fixed in formaldehyde and both barrier cells (13) and target (16) cells are subjected to further analysis. The real-time imaging in step 5 is used to identify possible drug candidates and target further analysis. Indirect immuno-fluorescence using monoclonal antibodies against epitopes that are critical indicators of drug efficacy or toxicity, to quantify and localise. Data is captured by confocal microscopy. This end-point analysis concentrates on the locations on the support where lead drug candidates have been achieved. If necessary, cells can be subjected to other forms of analysis whilst still on the support, for example electron microscopy. Further computational analysis is used to prioritise drug leads.

### Example 2 - Antibiotic Penetration Tests

To demonstrate an application for the invention in high content screening of antibiotic drugs designed to be taken orally then penetrate the intestine wall and kill a pathogenic bacterium within host macrophages.

A library of vancomycin-related glycopeptides with potential antibiotic activity targeted against the pathogenic bacterium *Salmonella typhimurium* is printed on the support and dried. Antibiotics (22) are printed as 300 micron diameter spots in gelatine or on support (21) with an altered surface chemistry to slow the drug release upon rehydration to a few hours. A 5 x 10 cm sheet of glass is used, printed with 5,000 drugs or drug combinations to create layer A (see figure 2).

A mammalian cell layer of Caco2 cells (13), simulating the intestinal barrier, is formed on a sheet of anopore support (support B) (10). Important characteristics of this layer are noted in Example 1.

Low-passage-number cultured J774 macrophages cells (16) are infected with S. *typhimurium* at levels designed cause > 90% macrophage cyotoxicity. The bacterially-infected macrophages are washed in phosphate buffered saline and any adherent extracellular bacteria are killed by an antimicrobial agent (26 micrograms/ml gentamycin). Gentamycin only kills extra-cellular bacteria (which may confound the assay) because of its poor penetration. The infected macrophages are recruited to the first surface ((11) Figure 1) of layer B (10) and Anopore^{™} (opposite to the Caco2 cells) by a biological adhesion matrix. The 0.2 micron pores of the Anopore^{™} prevents contamination of the barrier cells (13) with any bacteria that are released from dying cells, because bacteria are too large to pass through the pores.

Layers A (21) and B (10) are brought together, as shown in Figure 5 so that the barrier cells (13) are in contact with the antibiotic-printed layer (22). The entire array is incubated in mammalian growth medium in a CO₂ incubator at 37 °C for 18 hours. In this case recruitment of the macrophages to the first surface (11) is not sufficiently dense to block the access of nutrients to the Caco-2 cells. Each potential antibiotic spot will start to re-hydrate and penetrate the barrier cells directly or test the junctions between cells. The volume of medium above the target macrophage cells (16) is large and is re-circulating to ensure that antibiotics that are not taken up by the cells are dispersed.

At specific time periods (4 to 18 hours) the target macrophage cells are imaged and cytotoxicity determined using a fluorescent vital dye followed by fluorescent microscopy and imaging. Those regions where cytotoxicity is unusually low are good candidates for effective antibiotics that have preserved macrophage viability by their action on the intracellular *Salmonella.*

### REFERENCES

Goldstein and Betz, (1986) Sci Am. 255:74-83 "The blood-brain barrier"
Betz et al. (1994) J Cereb Blood Flow Metab. 14:29-37 "Blood-brain barrier permeability and brain concentration of sodium, potassium, and chloride during focal ischemia".
Sarah et al. (1997) J Pharmacol Exp Ther 281:1211-1218 "The Passage of Azido-deoxythymidine into and within the Central Nervous System: Does It Follow the Parent Compound, Thymidine?"
Nishino et al. (1997) J Pharmacol Exp Ther 282:1509-1517 "Transepithelial Transport of Organic Anions across the Choroid Plexus: Possible Involvement of Organic Anion Transporter and Multidrug Resistance-Associated Protein".
Pardridge, et al., (1992) West J Med. 156:281-286 "Blood-brain barrier and new approaches to brain drug delivery".
Pardridge (1992) Pharmacol Toxicol. 71:3-10 "Recent developments in peptide drug delivery to the brain".
Bickel, et al. (1993). Proc Natl Acad Sci USA. 90:2618-22 "Pharmacologic effects in vivo in brain by vector-mediated peptide drug delivery".
"Molecular Cloning: A Laboratory Manual" Second Edition (Sambrook et al,1989); "Animal Cell Culture" (R. I. Freshney, ed., 1987); the series
"Methods in Enzymology" (Academic Press, Inc.);
"Handbook of Experimental Immunology" (D. M. Weir & C. C. Blackwell, eds.);
"Gene Transfer Vectors for Mammalian Cells" (J. M. Miller & M. P. Calos, eds., 1987);
"Current Protocols in Molecular Biology" (F. M. Ausubel et al., eds., 1987, and periodic updtaes);
"Current Protocols in Immunology" (J. E. Coligan et al., eds., 1991).
Hafner (2000) Biosens. Bioelectron. 15:149-158 "Cytosensor Microphysiometer: technology and recent applications".
Gijon et al. (2000) J. Biol. Chem. 275:20146-20156 "Cytosolic phospholipase A2 is required for macrophage arachidonic acid release by agonists that Do and Do not mobilize calcium. Novel role of mitogen-activated protein kinase pathways in cytosolic phospholipase A2 regulation".
Checchelli et al (1999), Advanced Drug Delivery Reviews, 36: 165-178: "In vitro model for evaluating drug transport across the blood-brain barrier."

## Claims

1. Device for determining drug efficacy on target cells comprising to
(i) a porous support (B, 10) having first and second surfaces (11, 12) and at least one area with a plurality of through-going channels, wherein said area comprises on said first surface at least one region, which can be contacted with target cells (16), and said area comprises on said second surface (12) at least one region comprising a layer of barrier cells (13) presenting a significant barrier adhered to said second surface, wherein said region on the first surface is located opposite to said region on the second surface of the porous support (B, 10), and wherein said layer of barrier cells (13) provides a barrier for the passage of drugs; and,
(ii) a support (A, 21) comprising drugs (22) which can be contacted with the barrier cells (13) of the porous support (B, 10)

2. The device according to claim 1, wherein said barrier cells presenting a significant barrier are barrier cells forming tight junctions.

3. The device according to any of claims 1 or 2, wherein said support (A) comprising drugs is a porous support comprising drugs within the pores.

4. The device according to any of claims 1 to 3, further comprising target cells being in contact with a region on said first surface (11) of porous support (B).

5. The device according to claim 4, wherein said target cells are on a support (W, 31)

6. The device according to any of claims 3 to 5, wherein said barrier cells are sandwiched between porous support (B) and support (A).

7. The device according to any of claims 3 to 6, wherein said support (A) comprises a supply chamber, which is attached to said support (A) and wherein said supply chamber comprises at least one compartment.

8. Method for determining drug efficacy on target cells comprising:
(i) providing a porous support (B, 10) having first and second surfaces (11, 12) and at least one area with a plurality of through-going channels, wherein said area comprises on said first surface (11) at least one region, which can be contacted with target cells (16), and said area comprises on said second surface (12) at least one region comprising a layer of barrier cells (13) presenting a significant barrier adhered to said second surface, wherein said region on the first surface (11) is located opposite to said region on the second surface (12) of the porous support (B, 10), and wherein said layer of barrier cells (13) provides a barrier for the passage of drugs;
(ii) contacting said at least one region on said first surface of said porous support (B) with target cells except human embryonic stem cells,
(iii) contacting said layer of barrier cells on the second surface of the porous support (B) of said device with a support (A, 21) comprising drugs (22);
(iv) incubating said layer of barrier cells (13) with said drugs under conditions allowing the passage of said drugs through porous support (B, 10), and allowing binding of said drugs to target cells (16), and
(v) determining whether the drugs have bound to the target cells, whereby drug efficacy is determined.

9. Method according to claim 8, wherein said target cells are on the surface of a support (W) which is contacted with the first surface of said porous support (B).

10. Use of the device according to any of claims 1 to 7 for determining drug efficacy on target cells, components or fractions of target cells.

## Patentansprüche

1. Vorrichtung zur Bestimmung der Arzneimittelwirksamkeit, umfassend
(i) einen porösen Träger (B, 10), der eine erste und eine zweite Oberfläche (11, 12) und mindestens einen Bereich mit mehreren durchgehenden Kanälen aufweist, wobei der Bereich auf der ersten Oberfläche mindestens eine Region umfasst, die mit Zielzellen (16) in Kontakt gebracht werden kann und der Bereich auf der zweiten Oberfläche (12) mindestens eine Region umfasst, die eine Schicht aus eine Barriere ausbildende Zellen (13) enthält, wobei die Schicht eine wesentliche Barriere darstellt, die an der zweiten Oberfläche anhaftet, wobei die Region auf der ersten Oberfläche der Region auf der zweiten Oberfläche des porösen Trägers (B, 10) gegenüberliegend angeordnet ist und wobei die Schicht aus den eine Barriere ausbildenden Zellen (13) eine Barriere für den Durchtritt von Arzneimitteln darstellt; und
(ii) einen Träger (A, 21), der Arzneimittel (22) umfasst, die mit den eine Barriere ausbildenden Zellen (13) von dem porösen Träger (B, 10) in Kontakt gebracht werden können.

2. Vorrichtung nach Anspruch 1, wobei die eine Barriere ausbildenden Zellen, die eine wesentliche Barriere darstellen, eine Barriere ausbildende Zellen sind, die Tight Junctions bilden.

3. Vorrichtung nach einem der Ansprüche 1 oder 2, wobei der Träger (A), der Arzneimittel enthält, ein poröser Träger ist, der die Arzneimittel innerhalb der Poren enthält.

4. Vorrichtung nach einem der Ansprüche 1 bis 3, die ferner Zielzellen umfasst, die mit einer Region auf der ersten Oberfläche (11) des porösen Trägers (B) in Kontakt stehen.

5. Vorrichtung nach Anspruch 4, wobei die Zielzellen auf einem Träger (W, 31) sind.

6. Vorrichtung nach einem der Ansprüche 3 bis 5, wobei die eine Barriere ausbildenden Zellen sandwichartig zwischen dem porösen Träger (B) und dem Träger (A) eingeklemmt sind.

7. Vorrichtung nach einem der Ansprüche 3 bis 6, wobei der Träger (A) eine Versorgungskammer umfasst, die an dem Träger (A) befestigt ist und wobei die Versorgungskammer mindestens eine Abteilung enthält.

8. Verfahren zur Bestimmung der Arzneimittelwirksamkeit an Zielzellen, umfassend:
(i) Bereitstellen eines porösen Trägers (B, 10), der eine erste und eine zweite Oberfläche (11, 12) und mindestens einen Bereich mit mehreren durchgehenden Kanälen aufweist, wobei der Bereich auf der ersten Oberfläche (11) mindestens eine Region umfasst, die mit Zielzellen (16) in Kontakt gebracht werden kann und der Bereich auf der zweiten Oberfläche (12) mindestens eine Region umfasst, die eine Schicht aus eine Barriere ausbildende Zellen (13) enthält, wobei die Schicht eine wesentliche Barriere darstellt, die an der zweiten Oberfläche anhaftet, wobei die Region auf der ersten Oberfläche (11) der Region auf der zweiten Oberfläche (12) des porösen Trägers (B, 10) gegenüberliegend angeordnet ist und wobei die Schicht aus den eine Barriere ausbildenden Zellen (13) eine Barriere für den Durchtritt von Arzneimitteln darstellt;
(ii) Inkontaktbringen der mindestens einen Region auf der ersten Oberfläche von dem porösen Träger (B) mit den Zielzellen, mit Ausnahme von menschlichen embryonalen Stammzellen;
(iii)Inkontaktbringen der Schicht der eine Barriere ausbildenden Zellen auf der zweiten Oberfläche von dem porösen Träger (B) der Vorrichtung mit einem Träger (A, 21), der Arzneimittel (22)enthält;
(iv) Inkubieren der Schicht der eine Barriere ausbildenden Zellen (13) mit den Arzneimitteln unter Bedingungen, welche den Durchtritt der Arzneimittel durch den porösen Träger (B, 10) gestatten und die Bindung der Arzneimittel an die Zielzellen (16) ermöglichen; und
(v) Bestimmen, ob die Arzneimittel an die Zielzellen gebunden haben, wodurch die Arzneimittelwirksamkeit bestimmt wird.

9. Verfahren nach Anspruch 8, wobei sich die Zielzellen auf der Oberfläche von einem Träger (W) befinden, der mit der ersten Oberfläche von dem porösen Träger (B) in Kontakt gebracht wird.

10. Verwendung einer Vorrichtung nach einem der Ansprüche 1 bis 7 zur Bestimmung der Arzneimittelwirksamkeit an Zielzellen oder Komponenten oder Fraktionen von Zielzellen.

## Revendications

1. Dispositif destiné à déterminer l'efficacité de médicaments sur des cellules cibles, qui comprend :
(i) un support poreux (B, 10) ayant des première et deuxième surfaces (11, 12) et au moins une zone avec une pluralité de canaux traversants, ladite zone comprenant sur ladite première surface au moins une région, qui peut être mise en contact avec des cellules cibles (16), et ladite zone comprenant sur ladite deuxième surface (12) au moins une région comprenant une couche de cellules barrières (13) présentant une barrière significative collée à ladite deuxième surface, ladite région sur la première surface étant située à l'opposé de ladite région sur la deuxième surface du support poreux (B, 10), et ladite couche de cellules barrières (13) fournissant une barrière au passage de médicaments ; et,
(ii) un support (A, 21) comprenant des médicaments (22) et qui peut être mis en contact avec les cellules barrières (13) du support poreux (B, 10).

2. Dispositif selon la revendication 1, dans lequel lesdites cellules barrières présentant une barrière significative sont des cellules barrières formant des jonctions serrées.

3. Dispositif selon l'une quelconque des revendications 1 ou 2, dans lequel ledit support (A) comprenant des médicaments est un support poreux comprenant des médicaments à l'intérieur de ses pores.

4. Dispositif selon l'une quelconque des revendications 1 à 3, qui comprend en outre des cellules cibles en contact avec une région sur ladite première surface (11) du support poreux (B).

5. Dispositif selon la revendication 4, dans lequel lesdites cellules cibles se trouvent sur un support (W, 31).

6. Dispositif selon l'une quelconque des revendications 3 à 5, dans lequel lesdites cellules barrières sont prises en sandwich entre le support poreux (B) et le support (A).

7. Dispositif selon l'une quelconque des revendications 3 à 6, dans lequel ledit support (A) comprend une chambre d'alimentation, qui est fixée au dit support (A), la chambre d'alimentation comprenant au moins un compartiment.

8. Procédé de détermination de l'efficacité de médicaments sur des cellules cibles, qui comprend :
(i) la fourniture d'un support poreux (B, 10) ayant des première et deuxième surfaces (11, 12) et au moins une zone avec une pluralité de canaux traversants, ladite zone comprenant sur ladite première surface (11) au moins une région, qui peut être mise en contact avec des cellules cibles (16), et ladite zone comprenant sur ladite deuxième surface (12) au moins une région comprenant une couche de cellules barrières (13) présentant une barrière significative collée à ladite deuxième surface, ladite région sur la première surface (11) étant située à l'opposé de ladite région sur la deuxième surface (12) du support poreux (B, 10), et ladite couche de cellules barrières (18) fournissant une barrière au passage de médicaments ;
(ii) la mise en contact de ladite ou desdites régions sur ladite première surface dudit support poreux (B) avec des cellules cibles à l'exception de cellules souches embryonnaires humaines ;
(iii)la mise en contact de ladite couche de cellules barrières sur la deuxième surface du support poreux (B) dudit dispositif avec un support (A, 21) qui comprend des médicaments (22) ;
(iv) l'incubation de ladite couche de cellules barrières (13) avec lesdits médicaments dans des conditions permettant le passage desdits médicaments à travers le support poreux (B, 10), et le fait de permettre la liaison desdits médicaments aux cellules cibles (16), et
(v) la détermination de la liaison ou non des médicaments aux cellules cibles, permettant ainsi de déterminer l'efficacité médicamenteuse.

9. Procédé selon la revendication 8, dans lequel lesdites cellules cibles se trouvent sur la surface d'un support (W) qui est mis en contact avec la première surface dudit support poreux (B).

10. Utilisation du dispositif selon l'une quelconque des revendications 1 à 7, pour déterminer l'efficacité de médicaments sur des cellules cibles, des composants ou des fractions de cellules cibles.
